# EUROPEAN PATENT APPLICATION

(11) **EP 4 596 672 A1**
(43) Date of publication of application: **06.08.2025**
(21) Application number: 23872655.8
(22) Date of filing: 27.09.2023
(51) Int. Cl.: C12M 3/00, C12N 5/071, C12N 5/077, C12N 5/10

(54) **THREE-DIMENSIONAL TISSUE BODY AND METHOD FOR PRODUCING SAME**

(30) Priority: 28.09.2022 JP 2022155479
(71) Applicant: Saga University, Saga-shi, Saga 840-8502 (JP)
(72) Inventor: MURATA, Daiki, Saga-shi, Saga 840-8502 (JP); TAKAO, Shoko, Saga-shi, Saga 840-8502 (JP)
(74) Representative: J A Kemp LLP
(86) International application number: PCT/JP2023/036133
(87) International publication number: WO 2024/071436

(57) **Abstract**

A method for producing a three-dimensional tissue body, wherein the method comprises a step of culturing a three-dimensional structure formed by stacking cell masses, while applying stretch stimulation and/or compressive stimulation to the three-dimensional structure.

## Description

### Technical Field

The present invention relates to a three-dimensional tissue body and a method for producing the same, and particularly to a meniscus tissue body, etc. that is excellent in terms of biocompatibility, and a method for producing the same.

### Background Art

Injuries to the ligament, tendon, meniscus, etc. are often caused by sports, and are one of extremely frequently occurring orthopedic diseases. In particular, injuries to meniscus and anterior cruciate ligament (ACL) occur with high frequency.

As a current treatment for these injuries, an ACL reconstruction, for example, using autologous tendon grafts collected from the semitendinosus muscle, etc., has been mainly carried out. On the other hand, as an applied research into regenerative medicine, another ACL reconstruction is being performed by transplantation of a ligament-like structure formed using artificial biomaterials (synthetic fibers, collagen, etc.) without using autologous tendon grafts.

Recently, an attempt has been made to use a cell three-dimensional structure formed by fusing spheroids (cell masses) as a model of endocrine tissues or organs, or as materials for regenerative medicine (Patent Literature 1).

However, the tissues that develop orthopedic diseases are constantly subjected to repeated stresses of stretching (loading) and relaxation (unloading), and thus, it is difficult to reproduce the tissues treated by conventional treatment methods, and further, to allow these tissues to exhibit their functions.

For example, injury to meniscus tissues is one of extremely frequently occurring orthopedic diseases. In particular, the number of patients is increasing along with an increase in sports population. The meniscus, like the cartilage and tendon/ligament, has an extremely poor vascular distribution, and once damaged, the torn site does not naturally repair to its original tissue structure.

To date, meniscectomy has been performed for many years as a treatment for meniscus damage. Although discomfort and pain are temporarily relieved by resection of the meniscus, the function of the meniscus is significantly impaired even if the resection is partial. Furthermore, it is highly likely to provoke secondary knee osteoarthritis (OA).

For this reason, the current mainstream treatment is to preserve the meniscus as much as possible by suturing it. However, according to the estimate by the Ministry of Health, Labor and Welfare (NDB), the probability of re-rupture after suturing the meniscus is as high as 30%.

In addition, transplantation of a meniscus scaffold using biological and/or artificial materials is limited in the number of cases to which it is applicable, and there are also concerns about adverse events such as foreign body reactions and toxicity.

It is desired to develop a treatment method by which the repair results of the damaged site are maintained and/or improved, while preserving the meniscus as much as possible, and progression to OA is avoided.

As a technique capable of solving each of the above-described problems, a meniscus tissue body formed three-dimensionally using biological materials as raw materials does not require biological and/or artificial materials such as scaffolds, and has the same histological properties as the meniscus, and thus, many researchers are working on its novel synthesis.

For example, among tissue body forming devices that form tissue bodies by adding multiple cells into a culture vessel filled with a culture medium and then connecting the multiple cells to one another, a tissue body forming device has been known as a device for forming such a meniscus tissue body, wherein this tissue body forming device is formed of a frame body forming a hollow body having at least an opening unit at the top, wherein the frame body is housed in the culture vessel, and comprises a restricting frame unit that restricts diffusion of multiple cells, and a pressing and shaping unit that is disposed on the upper surface of the opening unit of the restricting frame unit and presses and shapes multiple cells housed in the opening unit (Patent Literature 2).

Moreover, there has been known a method for culturing animal cells, which is characterized in that it comprises immobilizing a culture carrier consisting of a plant-derived fibrous branched body in a culture vessel, and adhering animal cells to the culture carrier, followed by stretching and proliferation (Patent Literature 3).

### Citation List

### Patent Literature

Patent Literature 1: JP Patent Publication (Kokai) No. 2020-202785 A
Patent Literature 2: JP Patent Publication (Kokai) No. 2016-29 A
Patent Literature 3: JP Patent Publication (Kokai) No. 7-67626 A (1995)

### Summary of Invention

### Technical Problem

However, as in Patent Literatures 2 and 3, although it is likely to form a meniscus tissue body from a cell mass (spheroid) obtained by culturing multiple cells, an artificial meniscus tissue body capable of withstanding complicated and powerful mechanical loads, like the meniscus, has not yet been obtained.

Since the meniscus is located between the femur and the tibia of the knee joint, having frequent movements, the meniscus is constantly subjected not only to repeated stresses of stretching (loading) and relaxation (unloading), but also to compressive stimulation. Although this has not yet been elucidated in detail at present, it is known that the meniscus has a complex three-dimensional structure that has both the functions of tendons and ligaments and the functions of a cartilage. Accordingly, it is difficult to obtain a meniscus tissue body having a complicated structure that has both the functions of tendons and ligaments and the functions of a cartilage by the production methods described in Patent Literatures 2 and 3. In other words, a practical and strong meniscus tissue body that can function as a meniscus that can withstand both repeated stresses and compressive stimulation has not yet been obtained. In addition, such a meniscus tissue body that can function as a meniscus is highly desired because of the possibility of meniscus transplantation and its usefulness as a medical research model, but no such excellent meniscus tissue body is currently known.

Therefore, it has been desired to develop a medical material that can withstand physical strength and can be utilized in regenerative medicine.

### Solution to Problem

As a result of intensive studies directed towards achieving the aforementioned object, the present inventor has succeeded in obtaining a tissue body with an enhanced strength by applying stretch or compressive stimulation to a three-dimensional structure obtained by stacking cell masses, thereby leading to the present invention.

Specifically, the present invention is as follows.
[1] A method for enhancing the strength of a three-dimensional tissue body, comprising a step of culturing a three-dimensional tissue body formed by stacking cell masses, while applying stretch stimulation and/or compressive stimulation to the three-dimensional tissue body.
[2] The method according to the above [1], wherein the stretch stimulation is stretch stimulation including uniaxial movement or multiaxial movement.
[3] The method according to the above [2], wherein the stretch stimulation includes at least one movement from among twisting movement, tensile movement and vibration movement.
[4] The method according to the above [1], wherein the compressive stimulation is performed by applying pressure to the three-dimensional tissue body using a pressing unit having a pressing surface.
[5] The method according to the above [1], wherein a medium for culturing the three-dimensional tissue body further comprises an additive factor.
[6] The method according to the above [1], wherein the three-dimensional tissue body is cultured under circulating culture.
[7] The method according to the above [1], wherein the cell masses comprise mesenchymal stem cells, fibroblasts, and cells that are raw materials for a tissue body of interest.
[8] The method according to the above [7], wherein the cells that are raw materials are progenitor cells of bone, cartilage, ligament, tendon or meniscus.
[9] The method according to the above [8], wherein the progenitor cells are sclerotome cells.
[10] The method according to the above [9], wherein the three-dimensional tissue body is a tissue body for use in bone, cartilage, ligament, tendon or meniscus.
[11] A method for producing a three-dimensional tissue body with an enhanced strength, wherein the method comprises a step of culturing a three-dimensional tissue body formed by stacking cell masses, while applying stretch stimulation and/or compressive stimulation to the three-dimensional tissue body.
[12] The method according to the above [11], wherein the stretch stimulation is stretch stimulation including uniaxial movement or multiaxial movement.
[13] The method according to the above [11], wherein the stretch stimulation includes at least one movement from among twisting movement, tensile movement and vibration movement.
[14] The method according to the above [11], wherein the compressive stimulation is performed by applying pressure to the three-dimensional tissue body using a pressing unit having a pressing surface.
[15] The method according to the above [11], wherein a medium for culturing the three-dimensional tissue body further comprises an additive factor.
[16] The method according to the above [11], wherein the three-dimensional tissue body is cultured under circulating culture.
[17] The method according to the above [12], wherein the cell masses comprise mesenchymal stem cells, fibroblasts, and cells that are raw materials for a tissue body of interest.
[18] The method according to the above [17], wherein the cells that are raw materials are progenitor cells of bone, cartilage, ligament, tendon or meniscus.
[19] The method according to the above [18], wherein the progenitor cells of bone, cartilage, ligament, tendon or meniscus are sclerotome cells.
[20] The method according to the above [19], wherein the three-dimensional tissue body is a tissue body for use in bone, cartilage, ligament, tendon or meniscus.
[21] A three-dimensional tissue body having a breaking strength of at least 0.001 N/mm, which is prepared by culturing a three-dimensional tissue body formed by stacking cell masses, while applying stretch stimulation and/or compressive stimulation to the three-dimensional tissue body.
[22] The tissue body according to the above [21], wherein the cell masses comprise mesenchymal stem cells, fibroblasts, and cells that are raw materials for a tissue body of interest.
[23] The tissue body according to the above [22], wherein the cells that are raw materials are progenitor cells of bone, cartilage, ligament, tendon or meniscus.
[24] The tissue body according to the above [23], wherein the progenitor cells of bone, cartilage, ligament, tendon or meniscus are sclerotome cells.
[25] The tissue body according to the above [24], wherein the three-dimensional tissue body is a tissue body for use in bone, cartilage, ligament, tendon or meniscus.
[26] A culture device for culturing a three-dimensional tissue body, including an application unit for applying mechanical stimulation to the three-dimensional tissue body.
[27] The culture device according to the above [26], wherein
   the mechanical stimulation applied by the application unit includes at least stretch stimulation.
[28] The culture device according to the above [26], wherein
   the three-dimensional tissue body has an opening,
   the application unit includes at least two shaft members, and
   the application unit relatively moves the at least two shaft members in a state in which the at least two shaft members are inserted into the opening, so as to apply mechanical stimulation to the three-dimensional tissue body.
[29] The culture device according to the above [28], wherein
   the application unit relatively translationally moves the at least two shaft members, so as to apply mechanical stimulation to the three-dimensional tissue body.
[30] The culture device according to the above [28], wherein
   the application unit relatively rotationally moves the at least two shaft members, so as to apply mechanical stimulation to the three-dimensional tissue body.
[31] The culture device according to the above [26], wherein
   the application unit includes a vibrator that generates vibrations, and
   the application unit stretches the three-dimensional tissue body with the vibrations generated by the vibrator, so as to apply mechanical stimulation to the three-dimensional tissue body.
[32] The culture device according to the above [26], wherein
   the mechanical stimulation applied by the application unit includes at least compressive stimulation.
[33] The culture device according to the above [26], wherein
   the application unit includes
   a pedestal unit having a recess formed therein, into which the three-dimensional tissue body is fitted, and
   a pressing unit for pressing the three-dimensional tissue body fitted into the recess.
[34] The culture device according to the above [26], wherein
   the application unit includes
   a tensile unit for pulling the three-dimensional tissue body, and
   a pressing unit that presses the three-dimensional tissue body, to which stretch stimulation has been applied by the tensile unit, in a direction different from the tensile direction of the tensile unit.
[35] The culture device according to the above [26], which further includes
   a circulation unit for circulating a culture medium in a culture vessel.
[36] The culture device according to the above [26], which further includes
   a gas regulation unit for regulating gas in a culture vessel.
[37] The culture device according to the above [26], which further includes a temperature regulation unit for regulating temperature in a culture vessel.
[38] The culture device according to the above [26], which further includes
   a detection unit for detecting a physical quantity corresponding to the size of the mechanical stimulation applied to the three-dimensional tissue body by the application unit, and
   a control unit for controlling the size of the mechanical stimulation applied by the application unit, based on the detection results by the detection unit.

### Advantageous Effects of Invention

According to the present invention, there are provided a method for enhancing the strength of a three-dimensional tissue body, and a device used for the enhancing method. Such a tissue body is useful for the treatment of diseases in the field of orthopedics, or for regenerative medicine.

### Brief Description of Drawings

[Figure 1] Figure 1 is a view showing a configuration example of a culture device.
[Figure 2] Figures 2(a) to (d) include views explaining the movement of an application unit 1 and mechanical stimulation added to a three-dimensional tissue body 100 by the application unit 1.
[Figure 3] Figure 3 is a view showing an example of a mechanism for simultaneously performing a twisting movement and a tensile movement.
[Figure 4] Figure 4(a) is a view explaining the timing of a vibration movement, and Figures 4(b) and (c) are views explaining the position and order of giving vibrations.
[Figure 5] Figure 5 is a view showing a configuration example of a culture device.
[Figure 6] Figure 6 is a view showing a configuration example of a culture device.
[Figure 7] Figure 7 is a view showing differentiation of iPS cells into various tissues.
[Figure 8] Figure 8 is a flow chart showing a method for producing a three-dimensional tissue body according to an embodiment of the present invention.
[Figure 9] Figure 9 is a schematic view of a meniscus.
[Figure 10] Figure 10 is a view showing the immunohistochemical staining results of an artificial tissue body for ligament and tendon.
[Figure 11] Figure 11 is a view showing that a cell structure is placed in a device and is stretched to measure the strength of an artificial tissue body.
[Figure 12] Figure 12 is a view showing the tissues of an artificial tissue body for ligament and tendon, with and without stretch stimulation.
[Figure 13] Figure 13 is a view showing the imaging results of a polarized light microscopic image obtained by applying stretch stimulation (twist stretching) to an artificial tissue body for ligament and tendon, and subjecting the artificial tissue body to Picrosirius red staining.
[Figure 14] Figure 14 is a view showing the configuration of a circulating culture device in the culture step of an artificial tissue body.
[Figure 15] Figure 15 is a view showing the results of the breaking load of an artificial tissue body for ligament and tendon, with and without circulating culture in the culture step of the artificial tissue body.
[Figure 16] Figure 16 is a view showing the results of the rigidity of an artificial tissue body for ligament and tendon, with and without circulating culture in the culture step of the artificial tissue body.
[Figure 17] Figure 17 is a view showing the results of the breaking load of an artificial tissue body for ligament and tendon, according to the type of raw material cells in the culture step of the artificial tissue body.
[Figure 18] Figure 18 is a view showing the results of the breaking load and the optical microscopic images of an artificial tissue body for ligament and tendon, according to the type of raw material cells (the presence or absence of additive factors) in the culture step of the artificial tissue body.
[Figure 19] Figure 19 shows the polarized light microscopic images of a tissue section stained with Picrosirius red after transplantation of an artificial tissue body for ligament and tendon.
[Figure 20] Figure 20 shows the optical microscopic images of a tissue section stained with Picrosirius red after transplantation of an artificial tissue body for ligament and tendon.
[Figure 21] Figure 21 is a view showing the results obtained by stacking a cell aggregate on *Kenzan* (a needle-shaped product).
[Figure 22] Figure 22 is a view showing the results of the type I collagen immunostaining of a meniscus tissue body by an optical microscope.
[Figure 23] Figure 23 is a view showing the results of various types of staining of a meniscus tissue body.
[Figure 24] Figure 24 is a view showing ring-shaped and band-shaped artificial tissue bodies for meniscus.
[Figure 25] Figure 25 is a view showing one transplant for meniscus produced by bundling and suturing three band-shaped artificial tissue bodies for meniscus.
[Figure 26] Figure 26 is a view showing that the medial condyle of the femur of a pig was cut and inverted to expose the medial meniscus, which was then completely excised.
[Figure 27] Figure 27 is a view showing that a suture was passed through a bone hole created in the tibia and a transplant for meniscus was placed on the upper edge of the tibia.
[Figure 28] Figure 28 is a view showing the results of a fluoroscopic examination of a pig's knee joint four weeks after meniscus transplantation.
[Figure 29] Figure 29 is a view showing the results of a visual examination of a pig's knee joint when it was incised.
[Figure 30] Figure 30 is a view showing the results of a histological examination of a transplant for meniscus 4 weeks after the transplantation of meniscus.

### Description of Embodiments

The present invention relates to a method for enhancing the strength of a three-dimensional tissue body, comprising a step of culturing a three-dimensional tissue body formed by stacking cell masses, while applying stretch stimulation and/or compressive stimulation to the three-dimensional tissue body, and an enhanced three-dimensional tissue body.

The present invention is characterized in that a three-dimensional tissue body consisting of only cells is formed by stacking cell masses using a 3D bioprinter with a *Kenzan-*like needle-shaped body, and in that the three-dimensional tissue body is induced to differentiate into a tissue body with enhanced mechanical strength by making full use of the stretch culture device and compression culture device of the present invention. The present three-dimensional tissue body does not use artificial materials and has sufficient mechanical strength only with cells, which makes it possible to avoid injuries to various types of tissues and pathological progression for a long period of time.

### 1. Method for enhancing tissues and enhanced tissues

### (1) Cell masses

In the present invention, cell masses that constitute the three-dimensional tissue body comprise mesenchymal stem cells, fibroblasts, and cells that are raw materials for a tissue body of interest.

### (1-1) Mesenchymal stem cells

In the present invention, one of the components of the cell masses that constitute the three-dimensional tissue body is mesenchymal stem cells (MSCs). Since MSCs are pluripotent cells having self-replication ability and differentiation ability and have a low risk of tumor formation, MSCs can be expected to be a tool for cell therapy and regenerative medicine.

The origin of the MSCs used in the present invention is not limited, and examples of such origin may include fat, bone marrow, umbilical cord, pluripotent stem cells, and deciduous tooth pulp. In addition, MSCs are also commercially available, and can be obtained from ATCC, Evercyte, CellSource Co., Ltd., Gene Techno Science Co., Ltd. and the like.

Among the origins of the above-mentioned MSCs, pluripotent stem cells are stem cells that have the pluripotency capable of differentiating into all cells present in a living body and have the ability to proliferate, and examples of such pluripotent stem cells may include embryonic stem (ES) cells and induced pluripotent stem (iPS) cells. Preferred pluripotent stem cells are iPS cells.

ES cells are stem cells established from the cell masses of early embryos of mammals such as humans and mice. ES cells can be established by extracting the inner cell mass from the blastocyst of the fertilized egg of a target mammal and culturing the inner cell mass on fibroblast feeders. Moreover, human ES cell lines can be obtained from the Institute for Frontier Life and Medical Sciences, Kyoto University (Kyoto, Japan).

iPS cells are artificial stem cells derived from somatic cells and have pluripotency and self-proliferation ability that are almost the same as those of ES cells. The iPS cells can be produced by introducing specific reprogramming factors into somatic cells (Yamanaka S. et al., Cell, 126: 663-676, 2006; Okita K. et al., Nature 448, 2007; WO2007/069666, etc.). Genes contained in the reprogramming factors may include, for example, Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3 and Glis1. These reprogramming factors can be used alone or in an appropriate combination, but Oct3/4, Sox2, Klf4 and c-Myc are preferable.

The method of inducing iPS cells from somatic cells are well known (Yamanaka S. et al., Cell, 126: 663-676, 2006; Okita K. et al., Nature 448, 2007; WO2007/069666, etc.).

Examples of tissues, from which the iPS cells used in the present invention are derived, may include, for example, cartilage, bone, ligament, tendon, and meniscus.

In the present invention, the method of inducing mesenchymal stem cells (MSCs) from pluripotent stem cells may be, for example, a method of culturing iPS cells in the presence of a factor such as TGFB 1.

In addition, iPS cell-derived MSCs are also available from Kyoto University.

### (1-2) Fibroblasts

Fibroblasts are cells that constitute connective tissues, and fibroblasts produce dermal components such as collagen, elastin, and hyaluronic acid. In the present invention, for example, skin-derived fibroblasts (e.g., DFs) are used. In addition to the above-mentioned fibroblasts, other fibroblasts, such as, for example, stem cell-derived fibroblasts, can also be used in the present invention.

### (1-3) Cells that are raw materials for a tissue body of interest

In the present invention, the cells that are raw materials for a tissue body of interest are progenitor cells of bone, cartilage, ligament, tendon or meniscus, and examples of these cells are sclerotome cells or differentiated cells thereof.

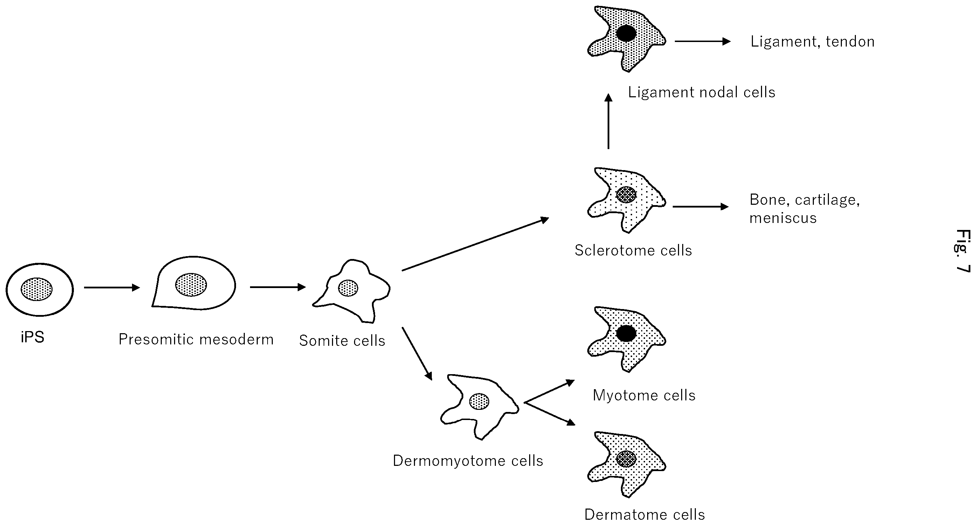

As shown in Figure 7, in the process of mesoderm differentiation, the presomitic mesoderm first differentiates into somite cells. Thereafter, the ventral part differentiates into sclerotome cells. Then, the sclerotome cells differentiate into cartilage, bone, or meniscus on the one hand, and into ligament nodal cells on the other hand. The ligament nodal cells further differentiate into ligaments and tendons. In the present invention, as progenitor cells of these cells, ligament nodal cells that are differentiated by one step from sclerotome cells can also be used, the origin of these cells may be iPS cells.

In the present invention, by using sclerotome cells, which are the source of mesodermal tissues, it is possible to allow the mesodermal tissues to differentiate into various mesodermal tissues.

### (2) Production of spheroid

In the present invention, first, mesenchymal stem cells, fibroblasts, and cells that are the raw material for the target tissue (hereinafter also referred to as "raw material cells") are mixed with one another to obtain a mixture. This mixture is formed as a cell mass (cell aggregate; spheroid), in which individual cells are mutually aggregated, for example, by mixing these three types of cells into a culture medium to prepare a cell suspension, and then culturing this suspension.

For example, when the cell mixture is cultured on a plate that has been treated to be water-repellent or non-adhesive to cells, such as, for example, a plate processed with Teflon (registered trademark), the cells seek a scaffold and adhere to each other to form a spheroid that is a cell aggregate. The culture time required until the spheroid is formed is 6 to 48 hours, and preferably 12 to 48 hours.

As another method of forming a spheroid, a container manufactured by IWAKI (EZsphere) can also be used, or a low-adhesive 10 cm or 6 cm dish manufactured by another company can also be used.

The culture medium for forming a spheroid is a standard culture medium that is commonly used for animal cell culture, and for example, Dulbecco's MEM medium (DMEM/High glucose), Dulbecco's MEM/Ham's F12 medium, RPMI-1640 medium, etc., can be used, and serum can be added to such medium.

It is to be noted that a spheroid does not need to be formed as an aggregate consisting of only the above three types of cells. As long as a spheroid is formed, multiple types of other cell species, such as undifferentiated cells such as umbilical cord blood-derived cells, or their differentiated-type cells, may be mixed in addition to the above three types of cells.

### (3) Stacking of spheroids

As described above, by further gathering the mixtures as cell masses and making them three-dimensional, a three-dimensional tissue body is formed as a scaffold-free cell structure having any shape (for example, strap-like (string-like)).

The cell masses can be processed into a three-dimensional body by using a known method. For example, according to the method described in Patent Document 1 or WO2008/123614, the cell masses are pierced into a needle-shaped product arranged in a *Kenzan* shape on a substrate, and the cell masses are arranged in any three-dimensional space, thereby stacking the cell masses to produce a three-dimensional structure body having a three-dimensional structure. Since an automatic stacking robot for realizing the above-described method has already been known (3D bioprinter "Regenova" (registered trademark), Cyfuse Co., Ltd.), the three-dimensional structure can also be produced using this robot. A three-dimensional structure is usually formed firmly in any shape, using cells and collagen exuded from the cells as main components.

The number and shape of the spheroids arranged are not particularly limited, and are arbitrary.

The ratio of the numbers of these mesenchymal stem cells, fibroblasts, and raw material cells is not particularly limited, and for example the ratio of mesenchymal stem cells : fibroblasts : raw material cells can be set to be 1 : 98 : 1 to 98 : 1 : 1 and 49 : 49 : 2 to 1 : 1 : 98. From the viewpoint of production efficiency, the ratio of raw material cells is preferably low, and the ratio of these cells can be set to be, for example, 49 : 49 : 2.

In one aspect of the present invention, this three-dimensional tissue body is a three-dimensional tissue body formed from iPS cell-derived mesenchymal stem cells, fibroblasts, and iPS cell-derived sclerotome cells. The ratio of the numbers of iPS cell-derived mesenchymal stem cells, fibroblasts, and iPS cell-derived sclerotome cells is also not particularly limited, and it can be set to be the same as the above-described ratio (IPS cell-derived mesenchymal stem cells: fibroblasts : iPS cell-derived sclerotome cells is 1 : 98 : 1 to 98 : 1 : 1 and 49 : 49 : 2 to 1 : 1 : 98). From the viewpoint of production efficiency, the ratio of the iPS cell-derived sclerotome cells is preferably low, and the ratio of these cells can be set to be, for example, 49 : 49 : 2.

### (4) Differentiation into individual tissue bodies, and three-dimensional tissue body

A three-dimensional structure obtained by stacking spheroids can be cultured in the presence of various differentiation factors to obtain a three-dimensional tissue body of individual tissues.

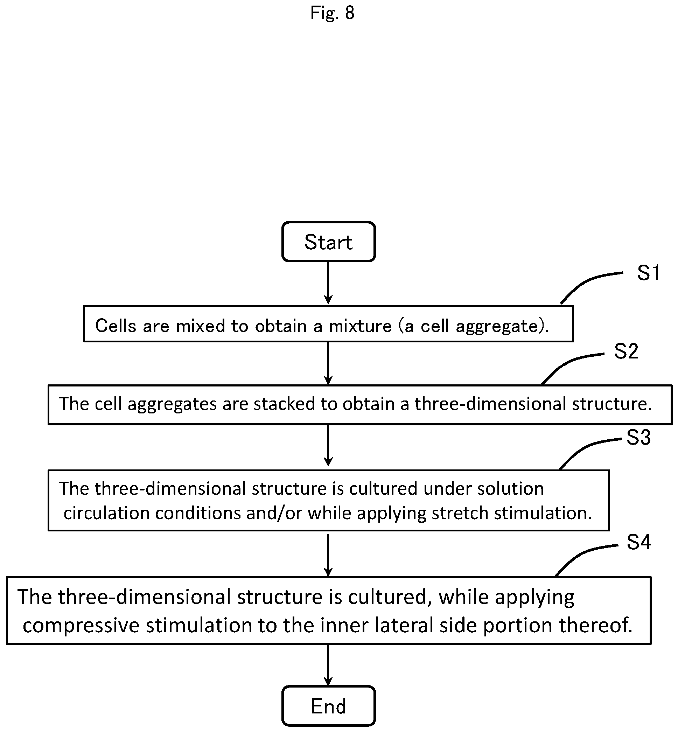

As an example of a method for producing a three-dimensional tissue body, as shown in Figure 8, first, mesenchymal stem cells (e.g., iPS cell-derived mesenchymal stem cells), fibroblasts, and raw material cells (e.g., iPS cell-derived sclerotome cells) are mixed with one another to obtain a mixture (S1: mixing step). This mixture is formed as a cell mass, in which these three types of cells are mutually aggregated, for example, by adding these three types of cells into a culture vessel filled with a culture medium and inducing a self-aggregation phenomenon.

This cell mass is formed by, but is not particularly limited to, gathering 30,000 to 40,000 cells. More preferably, various differentiation-inducing factors are further mixed into the culture vessel so as to form a mixture.

Next, the above cell mass is pierced into *Kenzan-*shaped needles and is stacked to form a three-dimensional structure (S2: stacking step). step).

Then, the above-described mixture is cultured in a culture vessel under solution circulation conditions and/or by applying stretch stimulation (S3: stretch step). The culture in this stretch step may be performed only under solution circulation conditions, only by applying stretch stimulation, or under solution circulation conditions and by applying stretch stimulation.

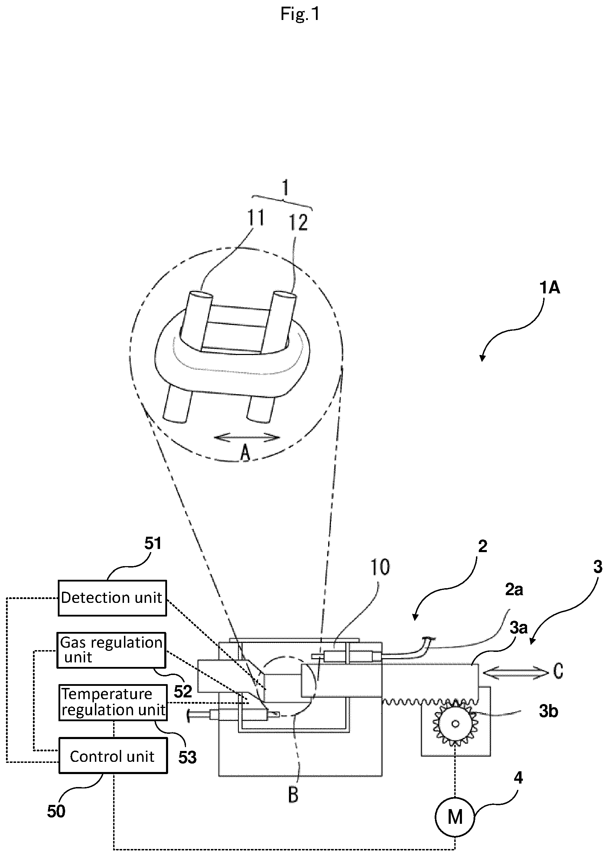

As an optimal and excellent culture vessel for easily realizing this stretch step, there is a culture vessel (also called an autostretching culture vessel) independently developed by the present inventors, as shown in Figure 1.

In one aspect of the present invention, compressive stimulation is optionally applied to the inner peripheral side of a three-dimensional structure, before, during, or after the stretch step, or throughout the entire process from before the stretch step to after the stretch step, and the three-dimensional structure is cultured, so that a three-dimensional tissue body of meniscus cells or the like can be obtained (S4: compression step).

It is to be noted that the stretch step and the compression step will be described in detail later.

Next, differentiation induction into individual tissues will be explained.

### (i) Bone

For differentiation into bone, additive factors such as BMP family, TGF family, FGF, IGF, HIF, and CTGF can be used, and for example, the cells are cultured in the presence of factors such as the BMP family. As a BMP family used for differentiation into bone, BMP-2, BMP-3, BMP-4, BMP-7 and BMP8a, etc. can be used alone or in an appropriate combination, but the BMP family are not limited thereto.

### (ii) Cartilage

For differentiation into cartilage, additive factors such as BMP family, TGF family, FGF, IGF, HIF, and CTGF can be used, as in the case of bone, and for example, the cells are cultured in the presence of factors such as the BMP family. As a BMP family used for differentiation into cartilage, BMP-2, BMP-5, BMP6, BMP-7 and BMP8a, etc. can be used alone or in an appropriate combination, but the BMP family are not limited thereto.

### (iii) Ligament or tendon

For differentiation into ligament or tendon, ligament or tendon differentiation-inducing factors can be used. As such ligament or tendon differentiation-inducing factors, BMP family, TGF family, FGF, IGF, HIF, and CTGF are known. Among these, example of the differentiation-inducing factors may include recombinant human TGF-β3 and growth differentiation factor 7 (human GDF-7), which can promote differentiation into ligament tendon cells and can mature the tissues to a stronger tissue structure. In addition, as BMP family members used for differentiation into ligament or tendon, BMP-12, BMP-13, BMP-14 and the like can be used alone or in an appropriate combination, but are not limited thereto.

This three-dimensional tissue body comprises ligament tendon cells differentiated from iPS cell-derived mesenchymal stem cells.

Moreover, this three-dimensional tissue body has a structure in which the orientation of type I collagen is aligned. The structure in which the orientation of type I collagen is aligned refers to a structure in which the tissues of type I collagen are oriented in a certain direction. This structure can be formed, for example, under solution circulation conditions and/or by applying stretch stimulation.

In the present invention, the artificial tissue body for ligament or tendon is composed of a ligament or a tendon formed from multiple sclerotome cells. The number of the sclerotome cells is not particularly limited, and it can be set to be, for example, 5 or more, 10 or more, 20 or more, 30 or more, 40 or more, or 50 or more.

### (v) Meniscus

The meniscus tissue body is more preferably formed by further comprising a meniscus differentiation-inducing factor. As such meniscus differentiation-inducing factors, BMP family, TGF family, FGF, IGF, HIF, and CTGF are known, and among these, for example, recombinant human TGF-β3 and BMP-12 can be used. These factors can promote differentiation of the meniscus tissue body into meniscus cells and can mature it to a stronger tissue structure. In other words, this three-dimensional tissue body comprises meniscus cells differentiated from iPS cell-derived mesenchymal stem cells. As a BMP family used for differentiation into meniscus, BMP-2, BMP-12, BMP-13, and BMP14 can be used alone or in an appropriate combination, but are not limited thereto.

The meniscus tissue body has a structure in which the orientation of type I collagen is aligned. The structure in which the orientation of type I collagen is aligned refers to a structure in which the tissue of type I collagen is oriented regularly, or in a certain direction to some extent. This structure in which the orientation is aligned can be formed, for example, under solution circulation conditions and/or by applying stretch stimulation.

Moreover, the meniscus tissue body has a structure in which the orientation of type II collagen is aligned. The structure in which the orientation of type II collagen is aligned refers to a structure in which the tissue of type II collagen is oriented regularly, or in a certain direction to some extent. This structure in which the orientation is aligned can be formed, for example, by applying compressive stimulation, as described in the production method described later. Thereby, an increase in proteins that hold water molecules is promoted in the structure part in which the orientation of type II collagen is aligned, making it possible to exhibit properties similar to those of cartilage. The increase in proteins that hold water molecules can be confirmed by safranin O staining (see the example described later).

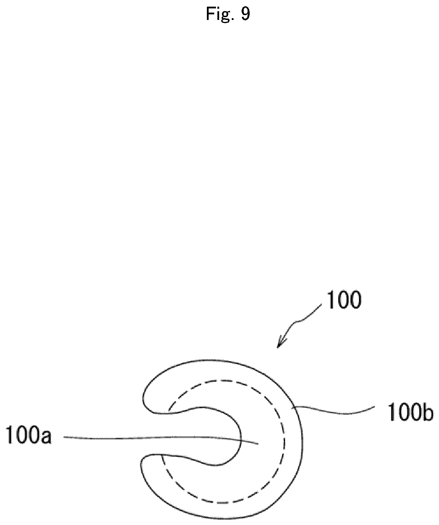

Regarding the relationship between the orientation of type I collagen and type II collagen, it is preferable that the orientation directions of type I collagen and type II collagen are aligned in mutually orthogonal orientations. Because of this characteristic orientation, as shown in Figure 9, in this three-dimensional tissue body 100, the orientation of type II collagen is aligned in an inner circumference lateral side portion 100a, and it has properties similar to those of cartilage, and in an outer circumference lateral side portion 100b, the orientation of type I collagen is aligned in a direction perpendicular to the orientation direction of this type II collagen, and it has properties similar to those of tendon and/or ligament, so that a meniscus tissue body having the structure actually possessed by meniscus can be obtained (see the examples described later).

Upon the formation of type I collagen and type II collagen, it is more preferable that stretch stimulation is applied to the entire mixture that is the source of this three-dimensional tissue body, and that compressive stimulation is then applied to the vicinity of the inner circumference lateral side portion of this mixture, followed by culturing it. By this stretch stimulation, first, the orientation of type I collagen is entirely aligned, and then, by compressive stimulation, the orientation of type II collagen is aligned only in the vicinity of the inner circumference lateral side portion. As a result, there is formed a three-dimensional tissue body, in which the orientation of type II collagen is aligned in the inner circumference lateral side portion of a meniscus tissue body and the orientation of type I collagen is aligned from inner circumference lateral side portion to the outer circumference lateral side portion.

Alternatively, a three-dimensional tissue body cultured while applying compressive stimulation and a three-dimensional tissue body cultured while applying stretch stimulation can be separately prepared. Then, the one with the properties of cartilage is disposed in the inner circumference lateral side portion and the one with the properties of tendon and/or ligament is disposed in the outer circumference lateral side portion, respectively, and they are then fused with each other, so that a meniscus tissue body can be provided.

In the present invention, the meniscus tissue body comprises meniscus formed from the multiple meniscus cells. The number of the meniscus cells is not particularly limited, and it can be set to be, for example, 5 or more, 10 or more, 20 or more, 30 or more, 40 or more, or 50 or more.

The meniscus tissue body according to the present embodiment can be widely utilized for various types of intended uses, and the use thereof is not particularly limited. For example, the present meniscus tissue body can be used for meniscus transplantation in the medical field, and it can also be utilized as a model capable of reproducing the functions of the meniscus for medical research.

### (5) Stretch step and/or compression step

In the present invention, in order to enhance the strength of a three-dimensional tissue body, the tissue body is cultured in a culture vessel, while applying stretch stimulation and/or compressive stimulation to the tissue body. In the culture in the stretch step and/or the compression step, the tissue body may be cultured only under solution circulation conditions, or may be cultured only by applying stretch and/or compressive stimulations. Otherwise, the tissue body may also be cultured under solution circulation conditions and by applying stretch and/or compressive stimulations.

Stretch stimulation includes uniaxial movement or multiaxial movement, and includes at least one movement from among twisting movement, tensile movement and vibration movement. On the other hand, compressive stimulation is performed by applying pressure to the three-dimensional tissue body, using a pressing unit having a pressing surface.

In addition to the above, with regard to a method for obtaining three-dimensional tissue bodies such as a meniscus tissue body, a tissue body cultured while applying compressive stimulation and a tissue body cultured while applying stretch stimulation can be separately prepared, and thereafter, the one with the properties of cartilage is disposed in the inner circumference lateral side portion and the one with the properties of tendon and/or ligament is disposed in the outer circumference lateral side portion, respectively, and they are then fused with each other, so that a meniscus tissue body can be provided.

### (5-1) Stretch stimulation

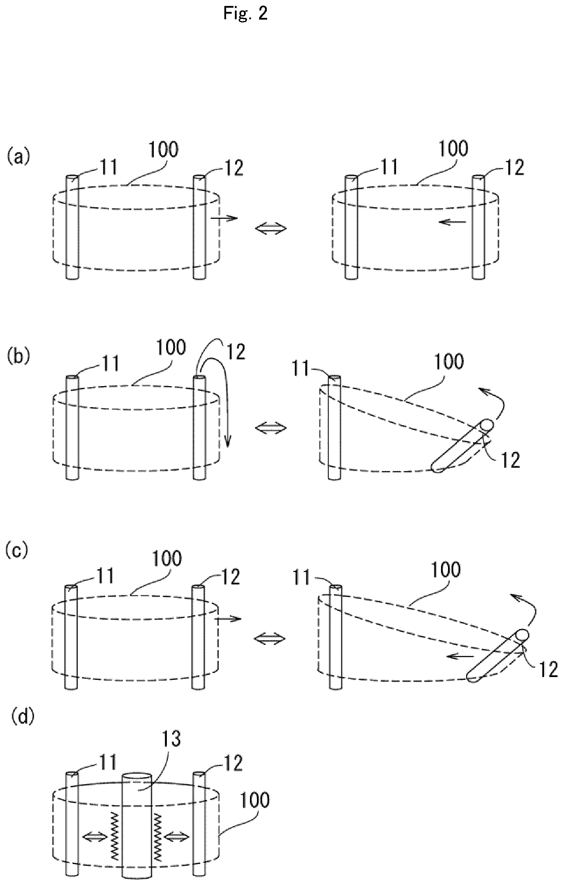

Details of an excellent culture device that is optimal for easily realizing the stretch step will be described later, but an overview of the stretch step will be explained using Figure 2.

As shown in Figure 2(a), uniaxial movement refers to a movement in which one opposing axis 11 and another opposing axis 12 stretch and contract a three-dimensional tissue body 100 (a tissue body formed in a ring shape) in a one-dimensional direction (linear direction). Also, multiaxial movement refers to a movement in which one opposing axis 11 and another opposing axis 12 stretch and contract a three-dimensional tissue body 100 in a two-dimensional direction (plane direction) or a three-dimensional direction (steric direction). For example, as shown in Figure 2(b), the multiaxial movement may include a twisting movement by at least one of one opposing axis 11 and another opposing axis 12. For example, the three-dimensional tissue body 100 can be stretched and contracted in a three-dimensional direction (steric direction) by a twisting movement in a three-dimensional direction by at least one of one opposing axis 11 and another opposing axis 12.

In the stretch step, the strength of the stretch stimulation is different depending on the type and size of the tissue body, and it is 0.001 to 10,000 [N] (1 [mN] to 10 [kN]) or 0.0001 to 1,000 [N/mm² (MPa)].

In addition, as shown in Figure 2(c), each of one opposing axis 11 and another opposing axis 12 can simultaneously include the above-described twisting movement and tensile movement. For example, by performing a two-dimensional twisting movement and a one-dimensional tensile movement by one opposing axis 11 and another opposing axis 12, respectively, as a result, the three-dimensional tissue body 100 can be stretched and contracted in a three-dimensional direction (steric direction). Also, for example, by combining the three-dimensional twisting movement and the one-dimensional tensile movement provided by one opposing axis 11 and another opposing axis 12, the three-dimensional tissue body 100 can be stretched and contracted in a more steric three-dimensional direction (steric direction).

Moreover, as shown in Figure 2(d), by allowing the three-dimensional tissue body 100 to include a vibrator 13 that generates vibrations, a vibration movement can be performed. Since this vibrator 13 generates vibrations in a three-dimensional direction (steric direction), the three-dimensional tissue body 100 can be stretched and contracted in the three-dimensional direction (steric direction). Moreover, by changing the vibration intensity of this vibrator 13, the strength of the stretching and contracting can be easily controlled.

In this way, the three-dimensional tissue body 100 that can adapt to repeated stresses of stretching (loading) and relaxation (unloading) is formed by the stretch stimulation including a uniaxial movement and/or a multiaxial movement.

The breaking load [mN] in the stretch step is 0.001 to 100,000 [N] (1 [mN] to 100 [kN]) or 0.0001 to 10,000 [N/mm² (MPa)].

In the present invention, various additive factors can be added before initiation of the stretch stimulation from the viewpoint of enhancing the strength of the tissue body, and appropriate cytokines can be added.

For example, addition of a bone morphogenetic protein (BMP) can promote formation of the meniscus itself, or cartilage tissues that constitute the meniscus. Examples of such bone morphogenetic proteins (BMPs) may include BMP-10, BMP-11, BMP-12, BMP-13, and BMP-14.

### (5-2) Compressive stimulation

Furthermore, in the present invention, compressive stimulation applied to the inner circumference lateral side portion of the tissue body during the stretch step, or before and/or after the stretch step, and the tissue body can be cultured.

Before initiation of this compressive stimulation, various additive factors can be added from the viewpoint of enhancing the strength of the tissue body, and appropriate cytokines can be added.

For example, addition of a bone morphogenetic protein (BMP) can promote formation of the meniscus itself, or cartilage tissues that constitute the meniscus. Examples of such bone morphogenetic proteins (BMPs) include BMP-10, BMP-11, BMP-12, BMP-13, and BMP-14.

The combination of additive factors and the combination of BMP families, for individual tissues, are the same as those described above.

However, the additive factors are not limited to the above combinations.

The compression load [mN] in the compression step is 0.001 to 100,000 [N] (1 [mN] to 100 [kN]) or 0.0001 to 10,000 [N/mm² (MPa)].

### (5-3) Properties of tissues caused by stretch stimulation and compressive stimulation

By the stretch stimulation, the orientation of type II collagen is aligned in the central portion of the three-dimensional tissue body (for example, the portion that constitutes the inner circumference lateral side portion of the meniscus tissue body), and at the same time, an increase in proteins that hold water molecules is promoted, which enables the tissue body to exhibit properties similar to those of cartilage. Thereby, the formation of a three-dimensional tissue body that can function as a shock absorber against various pressures. For example, when the three-dimensional tissue body is a meniscus tissue body, the inner circumference lateral side portion thereof can exhibit the cartilage function that is sufficient for the practical use of the meniscus.

### (6) Circulating culture

In the present invention, the strength of the three-dimensional tissue body can be enhanced by performing circulating culture using a circulating culture device.

The circulating culture is a culture performed during the stretch step of the three-dimensional tissue body, and refers to culturing by giving a flow to a medium in a culture vessel, including the flow of a medium in one direction and the convection of a medium within a vessel. The circulating culture is performed, for example, by connecting a peristaltic pump with a tube and allowing a culture medium to flow from the tube to the culture vessel. The medium that flows into the culture vessel flows through the vessel, is discharged from the vessel, and is used again for circulating culture.

For the circulating culture, it is recommended that 1 mL to 100 mL of medium flows into the culture vessel per unit time (minute), and that the same amount of medium is discharged.

Alternatively, the culture vessel can be shaken to create convection or vortexing of the medium.

### (7) Three-dimensional tissue body with enhanced strength

The thus produced three-dimensional tissue body is a structure composed only of cells without the need for a scaffold, and has higher mechanical strength than structures that have not been treated according to the present invention. The strength of the three-dimensional tissue of the present invention has a breaking strength (rigidity) of at least 0.001 N/mm, and has, for example, 0.001 to 100,000 [N] (1 [mN] to 100 [kN]).

The breaking load of each tissue when subjected to a stretching stimulus is 0.001 to 100,000 [N] (1 [mN] to 100 [kN]) or 0.0001 to 10,000 [N/mm] (MPa).

### 2. Culture device

Hereafter, a culture device for culturing the three-dimensional tissue body 100 will be described. In summary, the culture device comprises an application unit that applies a mechanical stimulus to the three-dimensional tissue body 100. By applying mechanical stimulation to the three-dimensional tissue body 100, the mechanical properties of the three-dimensional tissue structure 100 are improved.

Figure 1 is a view showing a configuration example of a culture device 1A as an example of the culture device. The culture device 1A includes a culture vessel 10, an application unit 1 composed of a pair of opposing axes 11 and 12 (axis members), a circulation unit 2 including a circulating culture tube 2a that circulates the culture medium, a reciprocating unit 3 that reciprocates by a motor, and a motor 4. In addition, the culture device 1A also includes a control unit 50, a detection unit 51, a gas regulation unit 52, and a temperature regulation unit 53.

The application unit 1 applies stretch stimulation to the three-dimensional tissue body 100 attached to the opposing axes 11 and 12 by the relative movement of the opposing axes 11 and 12 in the stretching direction A. Specifically, the three-dimensional tissue body 100 is first attached to the culture device 1A, so that the opposing axes 11, 12 are inserted into its opening. The opposing axis 12 is fixed to the reciprocating unit 3, and moves in a motor movable direction C in accordance with the movement of the reciprocating unit 3 to the motor movable direction C. In the present embodiment, since the stretching direction A and the motor movable direction C are parallel, this movement causes the opposing axis 12 to move relative to the opposing axis 11 in the stretching direction A. The movement of the opposing axis 12 away from the opposing axis 11 becomes a stretch movement to the three-dimensional tissue body 100, and thereby, stretch stimulation is applied to the three-dimensional tissue body 100. The application unit 1 can be configured to impart mechanical stimulation with a strength within the numerical value range (or a part thereof) described in the above section "1. Method for enhancing tissues and enhanced tissues, (6) Stretch stimulation and/or compressive stimulation."

In the present embodiment, the reciprocating unit 3 is a rack-and-pinion mechanism, and it has a movable unit 3a having a rack and to which the opposing axis 12 is fixed, and a connecting unit 3b having a pinion and being connected with the motor 4. However, the reciprocating unit 3 only needs to be able to relatively move the opposing axes 11 and 12, and other known techniques such as a ball screw mechanism can be appropriately used as a specific structure.

In the present embodiment, the detection unit 51 detects the magnitude of the mechanical stimulation imparted to the three-dimensional tissue body 100 by the application unit 1. The control unit 50 then controls the magnitude of the mechanical stimulation imparted by the application unit 1, based on the detection result of the detection unit 51. That is, the control unit 50 controls the magnitude of the mechanical stimulation imparted by the application unit 1 to approach a value of interest by feedback control.

The control unit 50 may include, for example, a microcomputer having a CPU (Central Processing Unit), ROM (Read Only Memory), RAM, input/output ports, etc., or various types of circuits. In addition, the detection unit 51 detects a physical quantity corresponding to the magnitude of mechanical stimulation (in the culture device 1A, the magnitude of stretch stimulation (traction force)). As such a detection unit 51, various types of sensors can be used, as appropriate. For example, the detection unit 51 may be a strain gauge type load cell, etc. Alternatively, the detection unit 51 may be, for example, a torque sensor that detects the torque of the motor 4, or may detect the drive current of the motor 4. In other words, it is sufficient for the detection unit 51 to be able to detect a physical quantity that directly or indirectly indicates the magnitude of the mechanical stimulation applied by the application unit 1. It is to be noted that, in the device 1A, the detection unit 51 detects the magnitude (traction force) of stretch stimulation as a magnitude of mechanical stimulation. However, the detection unit may detect compression force as a magnitude of mechanical stimulation, instead of traction force or in addition to traction force, depending on the configuration of the device.

In the present embodiment, the magnitude of mechanical stimulation imparted to the three-dimensional tissue body 100 by the application unit 1 is controlled based on the detection results of the detection unit 51, so that the stretch step can be effectively carried out. For example, as a method of controlling the operation of the application unit 1, a method of controlling the relative amount of movement of the opposing axes 11 and 12 is conceived. However, the hardness of the three-dimensional tissue body 100 cultured in the culture device 1A varies depending on the type, and the magnitude of the stretch stimulation imparted may differ even if the relative amount of movement of the opposing axes 11 and 12 is the same as each other. In addition, even when focusing on a specific three-dimensional tissue body 100, the hardness thereof may change over time if stretch stimulation is continued to be applied. In the present embodiment, the detection unit 51 provides feedback on the magnitude of mechanical stimulation, so that mechanical stimulation of an appropriate magnitude can be imparted to the three-dimensional tissue body 100.

Moreover, in the present embodiment, the culture device 1A includes a circulation unit 2 including a circulating culture tube 2a. Both ends of a flow path formed by the circulating culture tube 2a are connected to the culture vessel 10. Therefore, the medium in the culture vessel 10 is guided to the circulating culture tube 2a by a pump (not shown in the figures), and is then supplied to the culture vessel 10 again.

Furthermore, as an application example of the culture device 1A, a gas regulation unit 52 that regulates the gas in the culture vessel 10 can be further established, and oxygen gas can be injected into the culture vessel 10. This allows culture under various oxygen conditions, such as 1% oxygen or 20% oxygen. In addition, a temperature regulation unit 53 such as a heater that regulates the temperature in the culture vessel 10 may further established in the culture device 1A. This allows the culture vessel 10 to be maintained at a moderate temperature suitable for culture, making it possible to culture for a long period of time, and thereby, this culture vessel 10 itself can function as an incubator. In this way, while the circulating culture can be performed in an optimal environment, stretch stimulation can be provided.

The movement of the application unit 1 that provides stretch stimulation may be only uniaxial movement (translational movement in the stretching direction A) as described above, or may also include multiaxial movement. Herein, the multiaxial movement refers to movement with two or more movable axes. Examples of the multiaxial movement may include two-axis movement and three-axis movement. As the number of movable axes increases, it becomes possible to provide stretch stimulation to the three-dimensional tissue body 100 from various angles. For example, as described above, when the opposing axis 12 is connected with the motor 4, it is possible to configure that the opposing axis 12 performs uniaxial movement or multiaxial movement in conjunction with the motor 4. In addition, without being limited to this configuration, it is also possible for the opposing axis 11 to perform uniaxial movement or multiaxial movement, or it is also possible to configure that both the opposing axis 11 and the opposing axis 12 perform uniaxial movement or multiaxial movement.

An example of multiaxial movement will be explained. Figures 2(a) to 2(d) are views explaining the movement of the application unit 1 and the mechanical stimulation applied to the three-dimensional tissue body 100 thereby. Figure 2(a) shows the aforementioned uniaxial movement.

Figure 2(b) shows a case where the action of the application unit 1 includes a twisting movement. Herein, the action of the application unit 1 includes a twisting movement of the opposing axis 12. For example, the three-dimensional tissue body 100 can be stretched and contracted in a three-dimensional direction (steric direction) by a twisting movement in a three-dimensional direction by at least one of the opposing axes 11 and 12. The twisting movement can be imparted by, for example, the relative rotational movement of the opposing axes 11 and 12.

The twist stretching movement by this multiaxial movement aligns (lines up) the fibers themselves of the three-dimensional tissue body 100 in multiple directions, and as a result, more complex three-dimensional shapes such as ridges and uneven three-dimensional patterns along the longitudinal direction are easily formed. As histological findings, it has been confirmed that the characteristics similar to those of actual ligaments are well reproduced, making it possible to obtain a shape that is easily adapted to the tendons and muscles of the actual human body.

In addition, Figure 2(c) shows a case where the above-mentioned twisting movement and tensile movement are included at the same time. For example, the opposing axis 11 and the opposing axis 12 perform twisting movement in a two-dimensional and tensile movement in a one-dimensional direction, respectively, and as a result, stretching and contracting movement can be performed on the three-dimensional tissue body 100 in a three-dimensional direction (steric direction). Also, for example, by combining the above-mentioned three-dimensional twisting movement and one-dimensional tensile movement performed by the opposing axis 11 and the opposing axis 12, stretching and contracting movement can be performed on the three-dimensional tissue body 100 in a more steric three-dimensional direction (steric direction).

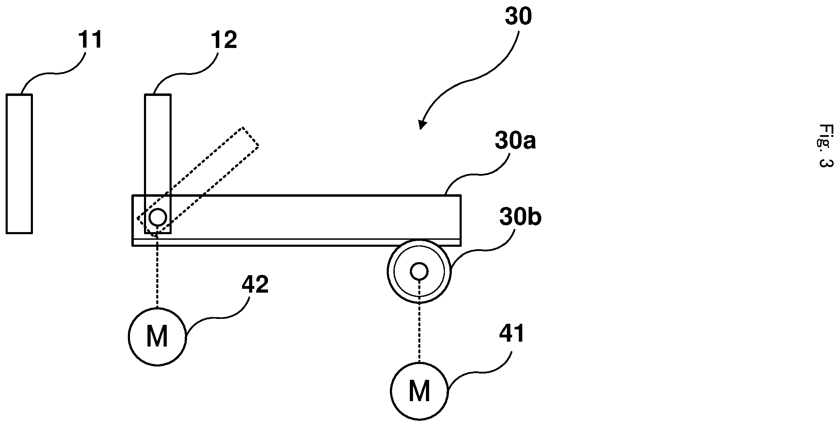

Figure 3 is a view showing an example of a mechanism for simultaneously performing twisting movement and tensile movement. Herein, the opposing axis 11 is fixed, and the opposing axis 12 is established in a mechanical unit 30. The mechanical unit 30 includes a movable unit 30a having a rack and on which the opposing axis 12 is established, and a connecting unit 30b having a pinion and being connected to a motor 41. The opposing axis 12 is established rotatably relative to the movable unit 30a by a motor 42. It is to be noted that the mechanical unit 30 is an example, and that any known technique capable of relatively translating and rotating the opposing axes 11 and 12 can be appropriately adopted. Moreover, the rotation direction of the opposing axis 12 is not limited to around one axis, and the opposing axis 12 may be relatively rotatable around multiple axes with respect to opposing axis 11. Furthermore, although opposing axis 11 is fixed herein, the opposing axis 11 may also be movable.

Also, Figure 2(d) shows a case where vibration movement is performed by including a vibrator 13 that generates vibrations. Since this vibrator 13 generates vibrations in a three-dimensional direction (steric direction), it is possible to stretch and contract the three-dimensional tissue body 100 in a three-dimensional direction (steric direction). In addition, by changing the strength of the vibrations of this vibrator 13, it is possible to easily control the strength of the stretching and contracting.

The vibrator 13 is not particularly limited as long as it vibrates, and for example, sonic vibration such as ultrasonic vibration or audible sound vibration can be used. Low-Intensity Pulsed Ultra Sound (LIPUS) can be used as such ultrasonic vibration, and in this case, low-output, gentle vibration is preferably applied to the three-dimensional tissue body 100.

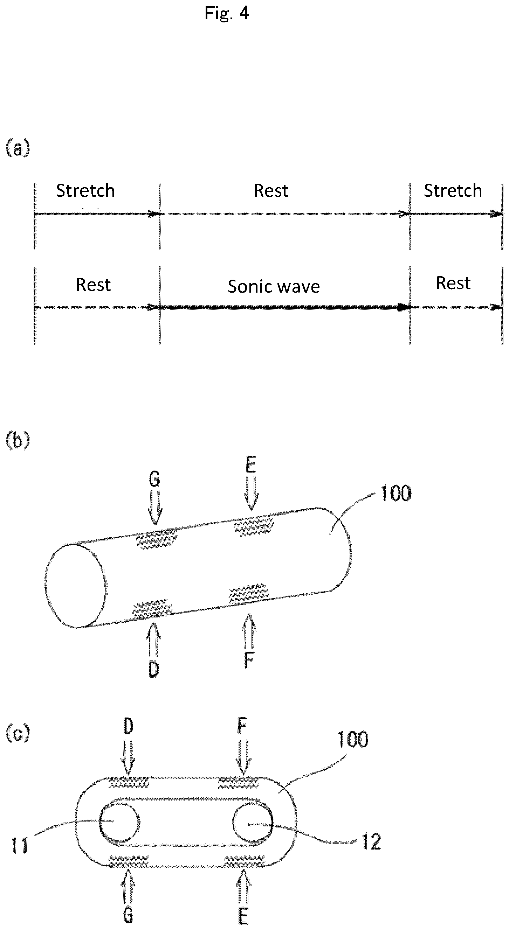

The timing of vibration generation by this vibrator 13 is not particularly limited, and as shown in Figure 4(a), vibration is preferably performed after the stretch movement has been applied to the three-dimensional tissue body 100 and during the rest period in which the stretch movement is stopped before the next stretch movement. For example, after a 30-minute stretch movement, sonic vibration is performed during a 23.5-hour rest period in which the stretch movement is stopped. This sonic vibration may be performed continuously or intermittently. After this rest period, the ultrasonic vibration is stopped and a 30-minute stretch movement is resumed. Thereafter, the same operation is repeated. In this manner, By cyclically repeating the stretch movement and the sonic vibration over time, the sonic vibration is applied in a state in which the stretch movement is stopped, and various types of vibrations are applied to the three-dimensional tissue body 100 in a complex and well-timed manner, which makes it possible to efficiently form a strong three-dimensional tissue body 100. In addition to this sonic vibration, it is also possible to use sound as a vibrator 13.

In addition, with regard to the vibration applied to the three-dimensional tissue body 100 by the vibrator 13, as shown in Figure 4(b) and Figure 4(c), different sites on the upper and lower surfaces of the three-dimensional tissue body 100 can be alternately vibrated in the order of an upper surface site D, a lower surface site E, an upper surface site F, and a lower surface site G. In other words, it is preferable that the upper surface sites D and F, and the lower surface sites E and G are different positions from each other.

Because of this vibration, as a three-dimensional movement, vibrations that are as if it were twisted is applied to the three-dimensional tissue body 100. In the culture step, a variety of complicated multiaxial movements that are composed of tensile movement (stretch movement), vibration movement and twisting movement are applied to the three-dimensional tissue body 100 in a continuous and multifaceted manner, so that a stronger three-dimensional tissue body 100 can be preferably formed.

It is to be noted that the above explanation is an example of alternating stretch movement and vibration movement, but the stretch movement and the vibration movement are not limited to this form, and it is also possible to simultaneously perform stretch movement and vibration movement on the three-dimensional tissue body 100.

For instance, in the early stage of the culture step, gentle vibration using, for example, low-intensity pulsed ultra sound (LIPUS), is applied to the three-dimensional tissue body 100 during the above-described rest period, and then, as the culture step progresses, it is possible to increase the vibration intensity or to perform stretch movement and vibration movement simultaneously without the rest period.

Thus, by increasing the intensity of the stretch movement and vibration movement over time, in the early stage of the culture step, gentle stimulation is applied to an immature and weak three-dimensional tissue body 100 without destroying the shape thereof, so that the base of the three-dimensional tissue body 100 can be strengthened. After that, from the middle to late stages of the culture step, stronger stimulation is applied through stretch and vibration movements to the three-dimensional tissue body 100 that has become more stable and mature in terms of maintaining its shape, thereby effectively enhancing the strength of the three-dimensional tissue body 100, so that the strength of the three-dimensional tissue body 100 can be effectively and stably increased.

In this way, by including uniaxial movement and/or multiaxial movement in the stretch stimulation, a three-dimensional tissue body 100 that can adapt to repeated stresses of stretching (loading) and relaxation (unloading) is formed, that is, an artificial tissue body for ligament/tendon that performs optimal functions for ligaments and tendons is reliably obtained.

For example, in the stretch step, the intensity of the uniaxial movement and/or multiaxial movement may be controlled based on the traction force applied to the three-dimensional tissue body 100. The traction force is a force applied to the three-dimensional tissue body 100 mainly by stretch stimulation.

In addition, for example, in the stretch step, when the traction force applied to the three-dimensional tissue body 100 becomes higher than a threshold, the intensity of the uniaxial movement and/or multiaxial movement may be controlled to be weakened. Also, for example, when the traction force applied to the three-dimensional tissue body 100 becomes lower than a threshold, the intensity of the uniaxial movement and/or multiaxial movement may be controlled to be strengthened.

Furthermore, after the above-described stretch step, compressive stimulation is applied to the inner circumference lateral side portion of the mixture, and the mixture is cultured to obtain a three-dimensional tissue body 100 containing meniscus cells.

Before initiation of this compressive stimulation, from the viewpoint of enhancing the strength of the tissue body, various types of additives can be added, for example, appropriate cytokines can be added. For instance, bone morphogenetic proteins (BMPs) can be added, which can promote formation of cartilage tissues that constitute the meniscus. Examples of such bone morphogenetic proteins (BMPs) are the same as those described above, and examples thereof may include BMP-10, BMP-11, BMP-12, BMP-13, and BMP-14. Of these, for example, BMP-12 can be added.

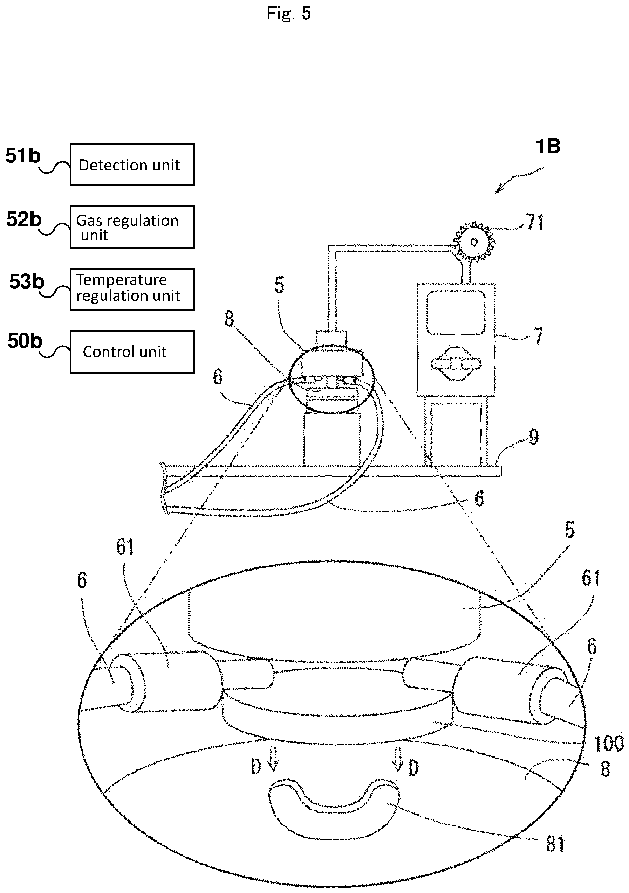

Figure 5 is a view showing a configuration example of a culture device 1B as an example of a culture device. The culture device 1B can be used to easily realize this compression step.

The culture device 1B includes a culture vessel 5 for culturing the mixture, a circulating culture tube 6 for circulating the culture medium, a tube tip unit 61 that is at the end of the circulating culture tube 6 and supplies the culture medium to the culture vessel 5, a motor control device 7 for controlling the compression of the mixture, a motor 71 controlled by the motor control device 7, a pedestal unit 8 for compression molding the mixture, and a machine base 9 for fixing the culture device 1B on a flat table.

As shown in the enlarged view of the circled portion in Figure 5, the culture vessel 5 connected to the motor 71 moves along the motor movable direction, thereby causing a compressive movement along the compression direction D, which is the direction in which the three-dimensional tissue body 100 is pressed down, and the central portion of the three-dimensional tissue body 100 (the portion constituting the inner circumference lateral side portion of the meniscus tissue body) is fitted into the structure installation groove 81 configured as a recess in the pedestal unit 8 and is then compressed, so that compressive stimulation can be accurately applied to the central portion of the three-dimensional tissue body 100. That is, in the culture device 1B, the culture vessel 5 and the pedestal unit 8 function as an application unit that applies compressive stimulation, which is an example of mechanical stimulation, to the three-dimensional tissue body 100.

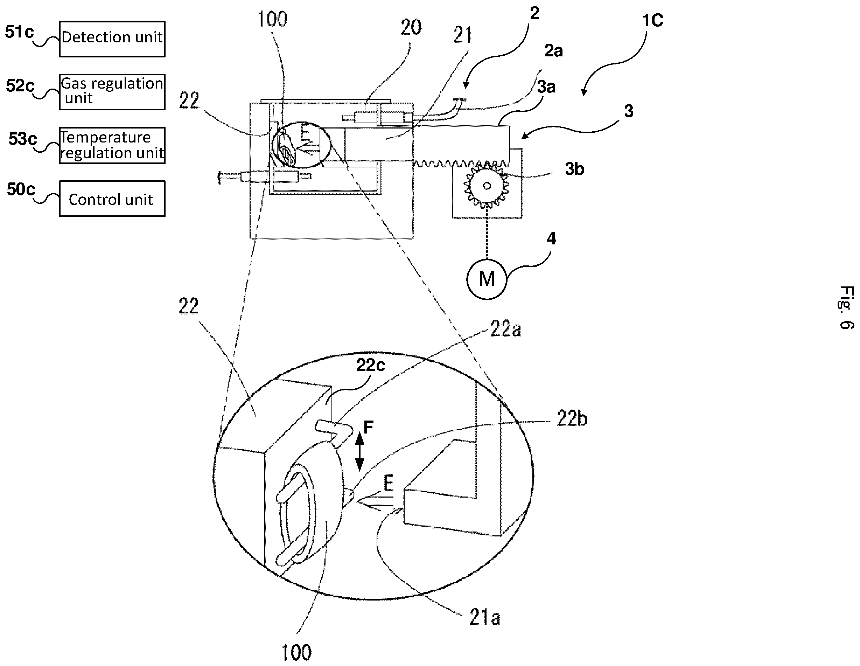

Figure 6 is a view showing a configuration example of a culture device 1C as an example of a culture device. The culture device 1C includes a pressing unit 21 that presses a three-dimensional tissue body 100 with a pressing surface 21a, and a fixing unit 22 having L-shaped hooks 22a and 22b that hook and fix a sheet-like or ring-like three-dimensional tissue body 100 in a stretched state. The culture device 1C includes a circulation unit 2 including a circulating culture tube 2a that circulates the culture medium, as in the culture device 1A shown in Figure 1, and a reciprocating unit 3 that is connected to a motor 4 that moves the pressing unit 21 in a straight line toward the fixing unit 22. As shown in the enlarged view of Figure 6, the three-dimensional tissue body 100 is hooked vertically to the two parallel-arranged L-shaped hooks 22a and 22b. In the culture device 1C, the sheet-like or ring-like three-dimensional tissue body 100, which is wound between these two hooks and hooked in a fully stretched state, is pressed against the wall 22c of the fixing unit 22 in the compression direction E by the pressing surface 21a. Thereby, it becomes possible to apply necessary and sufficient compressive stimulation to the three-dimensional tissue body 100. That is to say, in the culture device 1C, the pressing unit 21 and the fixing unit 22 function as an application unit that applies mechanical stimulation to the three-dimensional tissue body 100.

Herein, in the fixing unit 22, the L-shaped hooks 22a and 22b may be relatively movable in the stretching direction F. Thereby, compressive stimulation can be applied to the three-dimensional tissue body 100, while any stretch stimulation in the stretching direction F is applied to the three-dimensional tissue body 100. In other words, the culture device 1C may include a tensile unit (fixing unit 22) that pulls the three-dimensional tissue body 100, and a pressing unit 21 that presses the three-dimensional tissue body 100, to which stretch stimulation has been applied by the tensile unit, in a direction different from the tensile direction of the tensile unit. Moreover, by allowing the L-shaped hooks 22a and 22b to move relatively in the stretching direction F, the culture device 1C can be used for three-dimensional tissue bodies 100 of various sizes and shapes.

In addition, as a configuration corresponding to the control unit 50, the detection unit 51, the gas regulation unit 52 and the temperature regulation unit 53 possessed by the culture device 1A, the culture device 1B may include a control unit 50b, a detection unit 51b, a gas regulation unit 52b and a temperature regulation unit 53b. Also, as a configuration corresponding to the control unit 50, the detection unit 51, the gas regulation unit 52 and the temperature regulation unit 53 possessed by the culture device 1A, the culture device 1C may have a control unit 50c, a detection unit 51c, a gas regulation unit 52c and a temperature regulation unit 53c. In this case, the detection unit 51b may detect the magnitude of compressive stimulation as mechanical stimulation. Also, the detection unit 51c may detect the magnitude of stretch stimulation and/or compressive stimulation as mechanical stimulation.

### Examples

Hereinafter, the present invention will be more specifically described in the following examples. However, these examples are not intended to limit the scope of the present invention.

In the Examples, the following reagents, media, and cells were used.

### < Recombinant human fibroblast growth factor solution >

Recombinant human fibroblast growth factor (manufactured by Wako, 060-04543, 100 µg, Fibroblast Growth Factor Human recombinant) was dissolved in 0.1% BSA-PBS to prepare a 10 µg/ml rhFGF solution. Hereafter, this solution is referred to as an "FGF solution."

### < Recombinant human GDF-7 solution >

Recombinant Human GDF-7/BMP-12 (manufactured by R&D, 8386-G7-050, 50 µg, Growth Differentiation Factor-7) was dissolved in 4 mM hydrochloric acid to prepare a 500 µg/ml BMP-12 solution. Hereafter, this solution is referred to as a "BMP-12 solution."

### < Recombinant human CTGF solution >

Recombinant human CTGF (manufactured by R&D, 9190-CC-050, 50 µg, Connective Tissue Growth Factor) was dissolved in PBS to prepare a 500 µg/ml CTGF solution. Hereafter, this solution is referred to as a "CTGF solution."

### < Recombinant human TGF-β3 solution >

Recombinant Human TGF-β3 (manufactured by Lonza, PT-4124, 2 µg, Tissue Growth Factor-3) was dissolved in 4 mM hydrochloric acid to prepare a 20 µg/ml TGF-β3 solution. Hereafter, this solution is referred to as a "TGF-β3 solution."

### < Medium >

α-MEM manufactured by Nacalai Tesque, Inc., 21444-05 (supplemented with 10% FBS, 1% antibiotic, and 5 ng/ml FGF solution); fibroblast culture kit: FGM-2, manufactured by Lonza, CC-3132
Vascular endothelial cell culture kit: EGM-2, manufactured by Lonza, CC-3162
Structure production medium: FGM-2, manufactured by Lonza, CC-3132 and EGM-2, manufactured by Lonza; FBS was removed from CC-3162, and they were then mixed at a ratio of 1 : 1.

### < Cells >

Fibroblasts (DFs): manufactured by Lonza, CC-2509
iPS cell-derived mesenchymal stem cells (iPSC-MSCs): obtained from Kyoto University, a national university corporation
iPS cell-derived ligament tendon progenitor cells (iPSC-LPCs): obtained from Kyoto University, a national university corporation

### (Example 1) [Production of artificial tissues]

### (Example 1-1) Preparation of ligament and tendon tissue body

NHDFs were seeded on a 150 mm culture dish (FALCON, 353025) at a density of 8 x 10⁵ cells/dish, were then cultured for one week using a fibroblast culture kit, and were then harvested. iPSC-MSCs were seeded on a 150 mm culture dish (FALCON, 353025) at a density of 1 x 10⁶ cells/dish, were then cultured for one week in an α-MEM medium, and were then harvested. iPSC-LPCs were harvested on the 19th day after initiation of induction of ligament tendon progenitor cells from iPS cells.

The cells were mixed at a ratio of 10% iPSC-LPCs, 45% NHDFs, and 45% iPSC-MSCs, and were then suspended in a medium for structure production. Thereafter, the suspension was seeded in a 96-well plate (Sumitomo Bakelite Co., Ltd., MS-9096U) at a density of 3.5 x 10⁴ cells/well to form spheroids. Two days later, the spheroids were laminated on one another using a three-dimensional cell lamination system (Cyfuse, Regenova). Ten layers of spheroids were laminated on one another layered with intervals of 500 µm on a 7 mm diameter round *Kenzan* (Cyfuse, NA1029).

After culturing for seven days while circulating the medium for structure production with a peristaltic pump (registered trademark), the laminate was removed from the round *Kenzan* to obtain a circular three-dimensional tissue body. This peristaltic pump (registered trademark) is a tube pump (roller pump) that uses a roller to squeeze a tube such as silicone to deliver fluid.

Stretch stimulation was performed in the culture step of the three-dimensional tissue body. Two metal rods were inserted into the cavity of the circular three-dimensional tissue body, which was then placed in the device and was stretched. Stretching was initiated on the 7th day after the tissue body had been removal from the *Kenzan*, and the stretching was performed at a stretch percentage of 5%, a stretch number of 0.5 Hz, and a stretch time of 30 minutes per day. Stretch stimulation was applied for 5 consecutive days, followed by no stretching for 2 days, and this operation was repeated. Tendon ligament differentiation factors were added in the culture step of the three-dimensional tissue body. A BMP-12 solution, a CTGF solution, and a TGF-β3 solution were added at a concentration of 10 ng/ml each. The solutions were added one month after the tissue body had been removed from the *Kenzan*, and the obtained mixture was then cultured for one week.

### Immunohistochemical staining

The three-dimensional structure obtained above was fixed in 10% neutral buffered formalin, was then dehydrated and/or degreased, and was then thinly sectioned into paraffin blocks. As an antibody, rabbit anti-human MKX (ATLAS ANTIBODIES, HPA006927) or rabbit anti-human SCXA (abcam, ab58655) was used, and was reacted overnight at 4°C. Coloring was performed using a DAB polymer method.

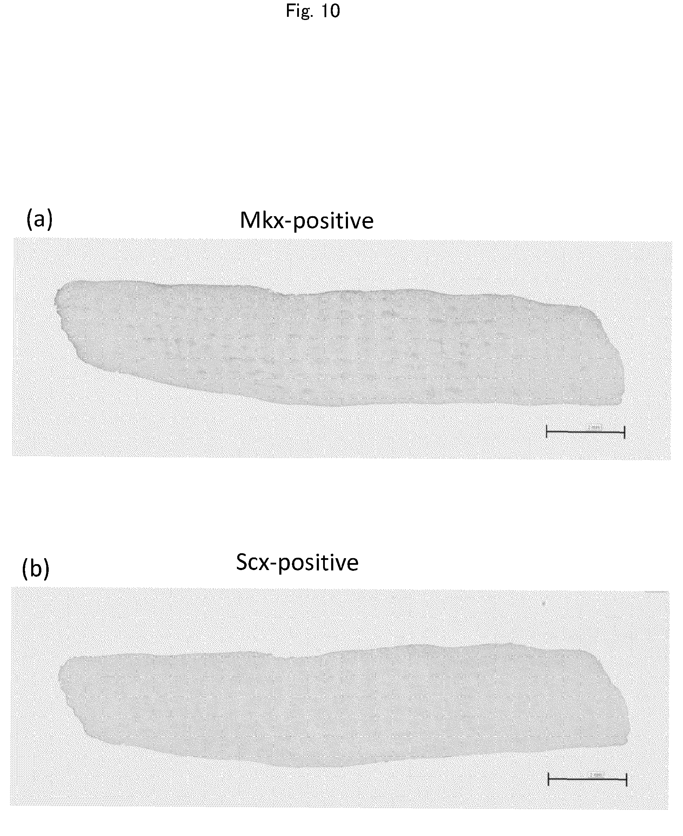

The obtained image results are shown in Figure 10. From the obtained results, Mkx-positive cells (Figure 10(a)) and Scx-positive cells (Figure 10(b)) were confirmed under the present experimental conditions, as shown in Figure 10, and furthermore, the presence of Tenm-positive cells was certainly confirmed. Thus, based on the description in the publication (Taiki Nakajima et al., Grafting of iPS cell-derived tenocytes promotes motor function recovery after Achilles tendon rupture, NATURE COMMUNICATIONS, 2021, https://doi.org/10.1038/s41467-021-25328-6), it was confirmed that the three-dimensional tissue body according to the present Example 1-1 was present as a ligament tendon.

### (Example 1-2) Confirmation of stretch stimulation

The presence or absence of stretch stimulation on the three-dimensional tissue body in the culture step was confirmed.

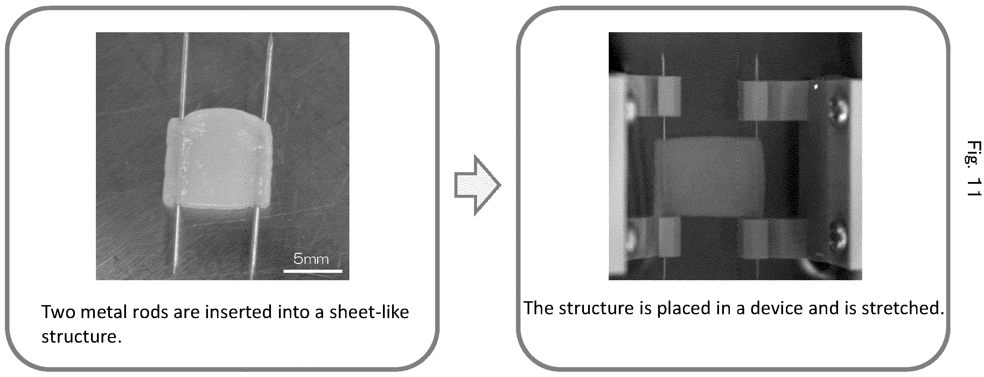

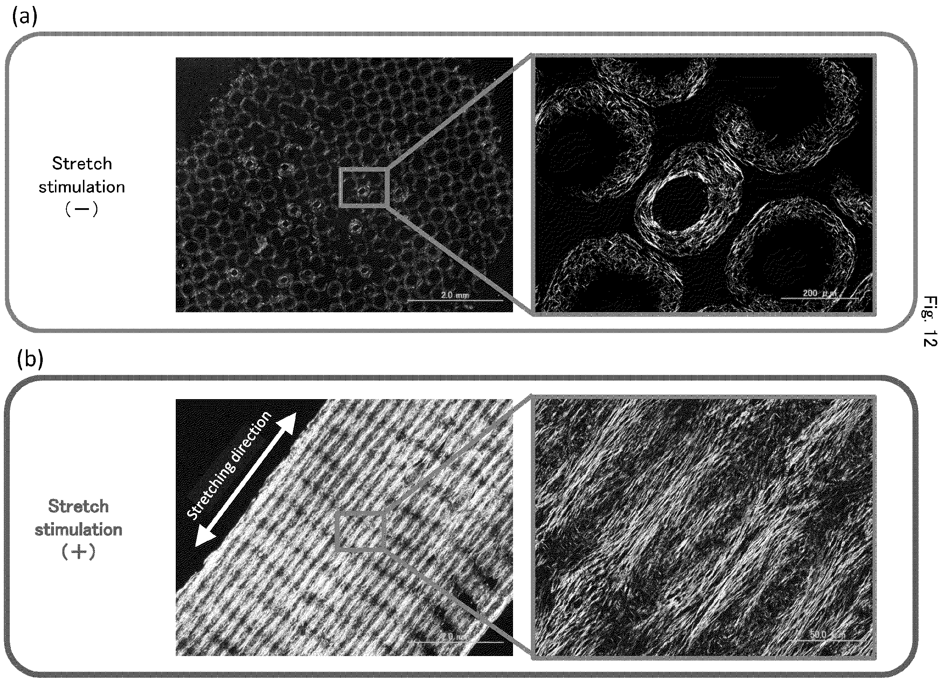

As shown in Figure 11, two metal rods serving as opposing axes 1 described above were inserted in parallel to each other into the lumen of the three-dimensional tissue body that was a tube-shaped structure, and the three-dimensional tissue body was then placed in the device (container) shown in Figure 1 and was stretched. The results of the images of the three-dimensional tissue body in the absence of stretch stimulation are shown in Figure 12(a). From the tensile test results shown in Figure 12(a), there was confirmed a structure, in which the orientation of type I collagen was not aligned and individual cell masses were randomly present. The results of the images of the three-dimensional tissue body in the presence of stretch stimulation are shown in Figure 12(b). From the results shown in Figure 12(b), there was confirmed a structure, in which the orientation of type I collagen was aligned in a certain direction along the stretch direction in the figure.

### (Example 1-3) Confirmation of stretch stimulation (twist stretching)

Since uniaxial movement was confirmed in the above Example 1-2, next, stretch stimulation by multiaxial movement was confirmed. Stretch stimulation (twist stretching) was performed on the three-dimensional tissue body in the culture step according to the same procedures as those in the above Example 1-2. Two metal rods serving as opposing axes 1 described above were inserted in parallel to each other into the lumen of the three-dimensional tissue body that was a tube-shaped structure, and the three-dimensional tissue body was then placed in the device (container) shown in Figure 1, and was stretched while twisting.

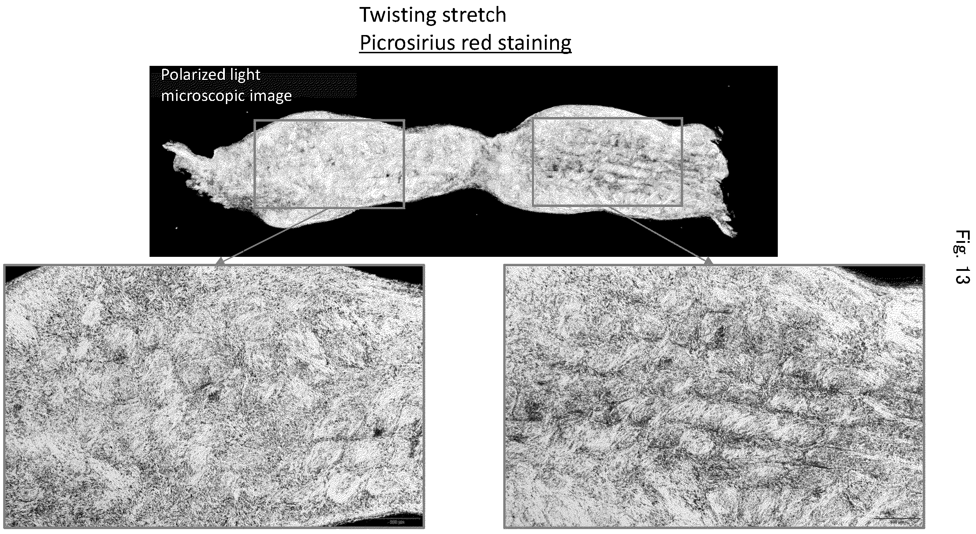

The three-dimensional tissue body after completion of the stretch stimulation was subjected to Picrosirius red staining (Picro-Sirius Red Stain), and the collagen fibers on tissue sections were stained. A polarized light microscopic image of the three-dimensional tissue body after completion of the Picrosirius red staining is shown in Figure 13(a). The left portion and the right portion of the three-dimensional tissue body are enlarged and are shown in Figures 13(b) and (c), respectively.

From the results of the obtained polarized light microscopic images, it was confirmed that the brightly lighted portion was reduced compared to the results shown in Figure 10 obtained in Example 1-2. In general, the more brightly lighted portion there are in a polarized light microscopic image, the stronger the tendency for unidirectionality. Thus, it was confirmed that the collagen fibers were aligned in multiple axial directions by the twist stretching caused by the multiaxial movement of the present example, while a certain degree of unidirectionality is maintained in this three-dimensional tissue body as a whole.

In addition, as shown in Figure 13, in the three-dimensional tissue body to which twist stretching was applied by this multiaxial movement, more steric complicated shape, such as ridges and uneven three-dimensional patterns along the longitudinal direction, were formed, and as histological findings, it was confirmed that the characteristics of actual ligaments were well reproduced.

### (Example 1-4) Confirmation of circulating culture

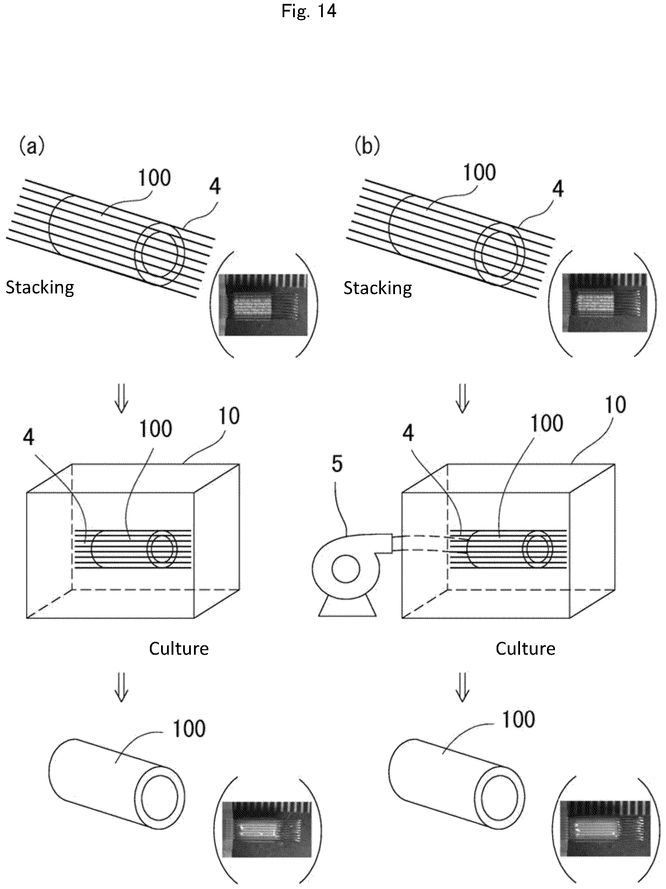

According to the same procedures as those in the above Example 1-1, the three-dimensional tissue body in the culture step was confirmed in the presence or absence of circulating culture. As shown in Figure 14, after cell masses had been laminated in a tubular shape on a Kenzan 4, the laminate was placed in the device (vessel) shown in Figure 1, and a tensile test was performed to confirm a difference in the presence or absence of a tube pump 5, namely, a difference between the absence of the tube pump 5 such as a peristaltic pump (registered trademark) that enables circulating culture in the vessel (static culture) as shown in Figure 14(a), and the presence of the peristaltic pump connecting to a tube (circulating culture) as shown in Figure 14(b). This *Kenzan* 4 is a *Kenzan* approximately 3.4 mm square with 9 needles each arranged vertically and horizontally with intervals of 400 µm.

### Tensile test

A force gauge (Imada, ZTA-500N) was attached to a motorized measuring stand (Imada, MX2-500N-FA), and the measurement was carried out. Two metal rods were passed through the lumen of the three-dimensional tissue body, one of which was fixed to the stand, and the other was passed through the force gauge. The force gauge side was pulled at a speed of 50 mm/min, and the load at which the three-dimensional tissue body was broken was examined.

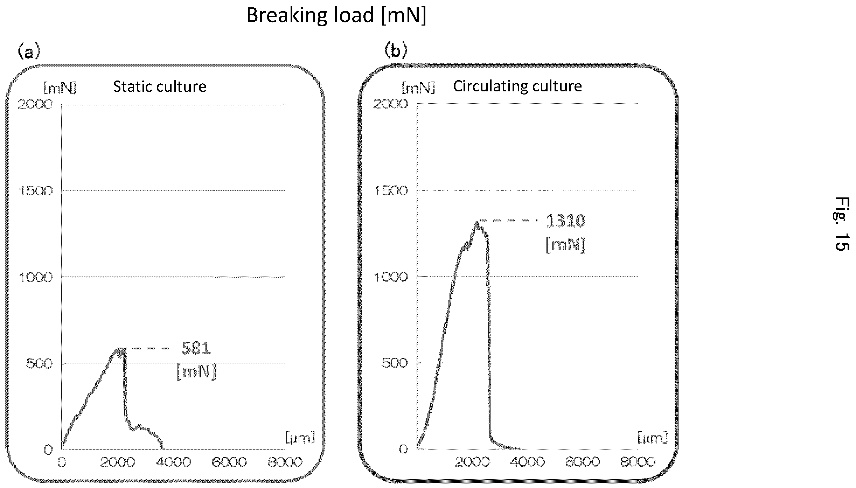

Regarding breaking load [mN], the results of the static culture for the breaking load [mN] are shown in Figure 15(a), whereas those of circulating culture for breaking load [mN] are shown in Figure 15(b). From the results of Figure 15, it was confirmed that the circulating culture (1310 [mN]) exhibited two or more times the strength (i.e., the breaking load [mN]) than the static culture (581 [mN]).

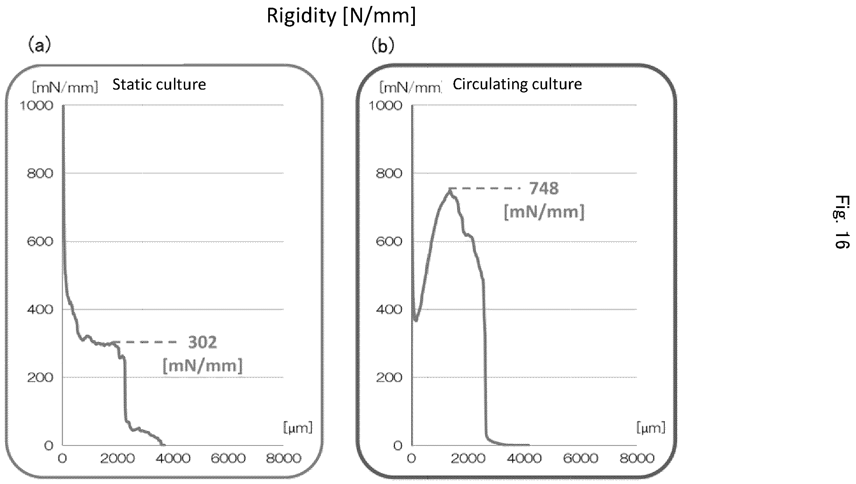

Moreover, regarding rigidity [N/mm] as well, the results of static culture for rigidity [N/mm] are shown in Figure 16(a), whereas those of circulating culture for rigidity [N/mm] are shown in Figure 16(b). From the results of Figure 16, it was confirmed that the rigidity [N/mm] of the circulating culture (748 [mN/mm]) is two or more times the strength (i.e., the breaking load [mN]) than that of the static culture (302 [mN/mm]).

### (Example 1-5) Confirmation of raw material cells

According to the same procedures as those of the above Example 1-1, the types of raw material cells in the mixing step were confirmed.

Two types of three-dimensional tissue bodies (artificial tissue bodies for ligament/tendon) were examined; namely, a case where iPS cell-derived mesenchymal stem cells were used as raw material cells with respect to fibroblasts, and a case where somatic stem cells were used as raw material cells.

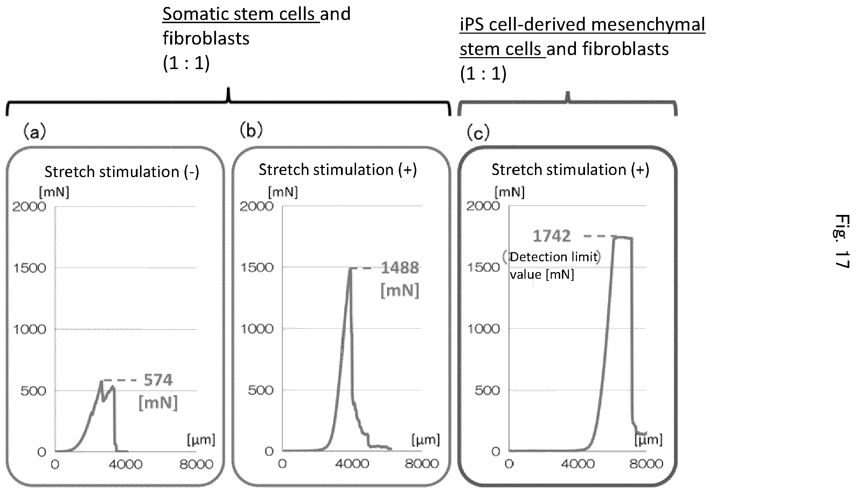

Regarding breaking load [mN], the results of a case where fibroblasts and somatic stem cells were used (with stretch stimulation) are shown in Figure 17(b). The composition ratio between the fibroblasts and the somatic stem cells was 1 : 1. In addition, the results of a case where fibroblasts and somatic stem cells were used (without stretch stimulation) are shown in Figure 17(a). The composition ratio between the fibroblasts and the somatic stem cells was 1 : 1. Moreover, the results of a case where fibroblasts and iPS cell-derived mesenchymal stem cells were used (with stretch stimulation) are shown in Figure 17(c). The composition ratio between the fibroblasts and the iPS cell-derived mesenchymal stem cells was 1 : 1.

From the results shown in Figure 17, it was confirmed that when the fibroblasts and the iPS cell-derived mesenchymal stem cells were used (with stretch stimulation), the breaking load [mN] exceeded the detection limit value, and a stronger strength was obtained in the use of the fibroblasts and the iPS cell-derived mesenchymal stem cells than when the fibroblasts and the somatic stem cells were used.

### (Example 1-6) Confirmation of raw material cells (presence or absence of additive factors)

According to the same procedures as those of the above Example 1, a difference in the types of raw material cells in the mixing step (the presence or absence of additive factors) was confirmed. That is to say, among the raw material cells, the composition ratio of fibroblasts and iPS cell-derived mesenchymal stem cells was set to be 1 : 1, and three-dimensional tissue bodies (artificial tissue bodies for ligament/tendon), which were obtained, with and without addition of the aforementioned BMP-12, CTGF and recombinant human TGF-β3 as additive factors, were pulled.

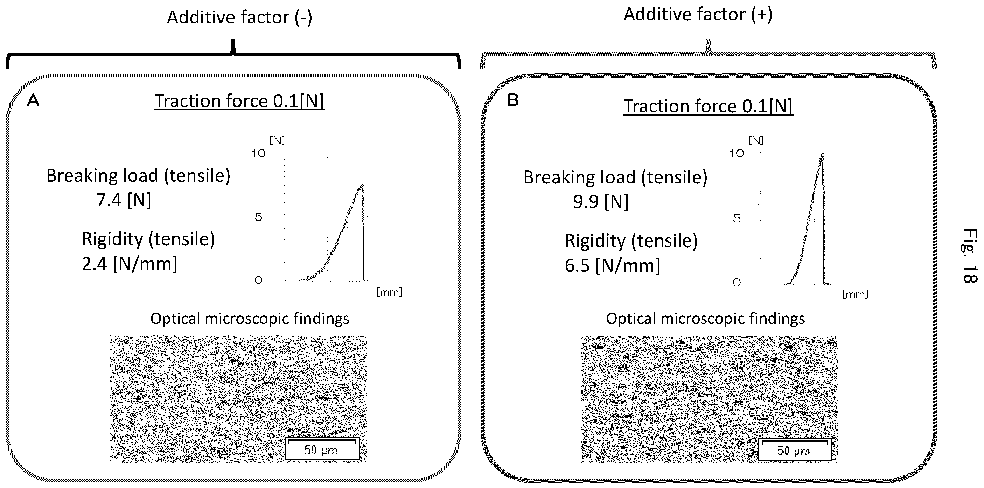

The traction results of the three-dimensional tissue body obtained without addition of the additive factors are shown in Figure 18(a). In addition, the traction results of the three-dimensional tissue body obtained with addition of the additive factor are shown in Figure 18(b).

From the results shown in Figure 18, it was found that the three-dimensional tissue body obtained with addition of the additive factors had high values, namely, a breaking load of 9.9 [N] and a rigidity of 6.5 [N/mm]. When these values were converted to the size of a tissue body to be transplanted into rabbits, the values were 23.8 N and 15.5 N/mm. These results show that the three-dimensional tissue body obtained with addition of the additive factors had strong strength, such that the breaking load of the three-dimensional tissue body obtained with addition of the additive factors was about 1.5 times higher and the rigidity thereof was about 3 times higher than the three-dimensional tissue body obtained without addition of the additive factors. Moreover, from the optical microscopic findings, it was confirmed that the collagen fibers in the three-dimensional tissue body obtained with addition of the additive factors were thicker, more abundantly distributed, and more aligned in one direction in terms of orientation, than the three-dimensional tissue body obtained without addition of the additive factors.

### (Example 1-7) Immunodeficient miniature pig model

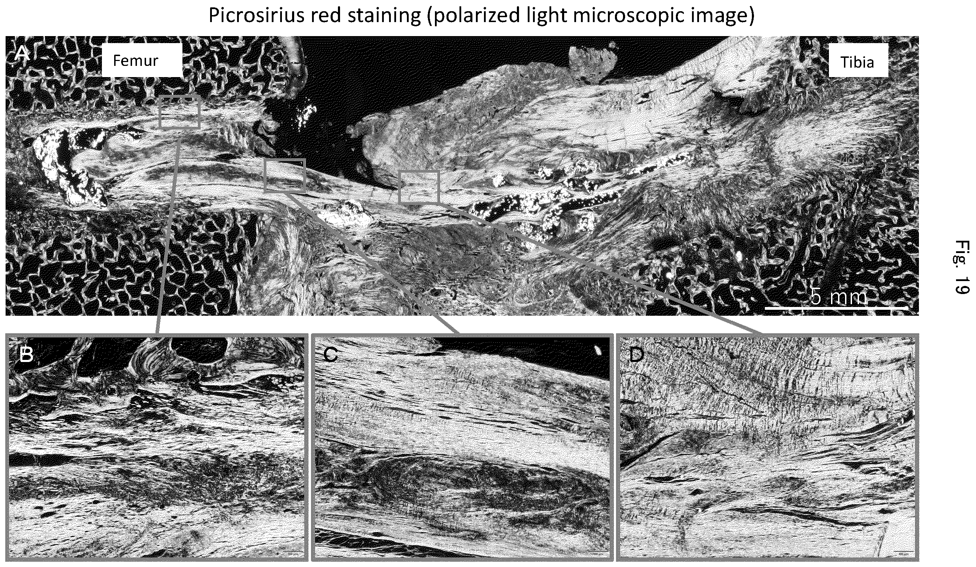

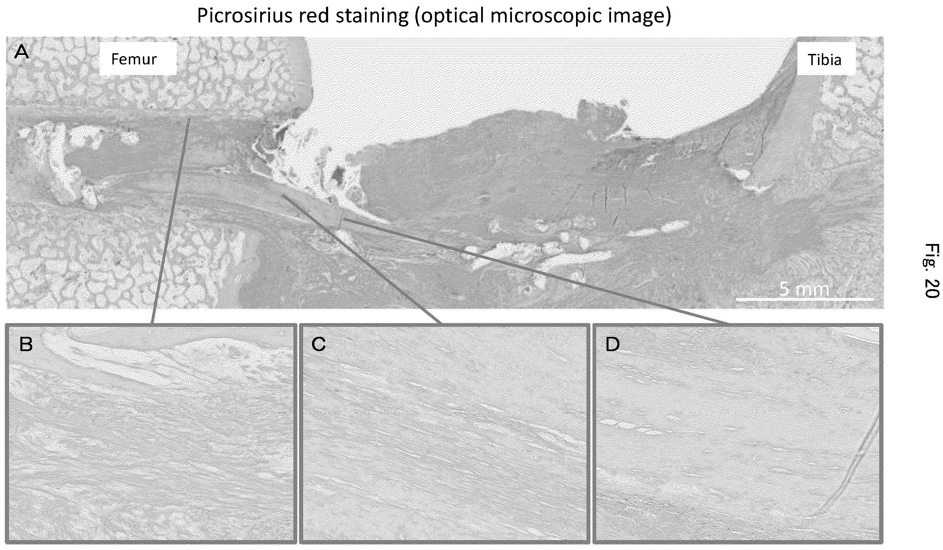

An immunodeficient miniature pig model [Itho et al. Nat Commun. 2019] was also created, and the three-dimensional tissue body (artificial tissue body for ligament/tendon) obtained in the above Example 1-6 above was transplanted therein. In order to confirm the histological characteristics of the three-dimensional tissue body (artificial tissue body for ligament/tendon) transplanted as a reconstituted ligament into this immunodeficient miniature pig, the tissue sections after the transplantation were subjected to Picrosirius red staining. The results of the tissue sections stained with Picrosirius red, which were observed by polarizing and optical microscopy, are shown in Figure 19 and Figure 20, respectively.

From the obtained results, it was found that the transplanted artificial tissue body for ligament/tendon had already engrafted at the time point of one month after the transplantation, and that numerous Sharpey's fibers had been formed at the attachment site between the artificial tissue body for ligament/tendon and the bone, and early fusion had been observed. Thus, it was confirmed that the adhesion between the artificial tissue body for ligament/tendon and the bone had significantly progressed. Moreover, at the time point of one month after the transplantation, the transplanted artificial tissue body for ligament/tendon had matured into a ligament *in vivo*, and the collagen fibers of the transplant were very thick and bundled, very closely resembling the image of the original ligament tissues. Furthermore, at the time point of one month after the transplantation, it was confirmed that the adhesion between the tissue body and the residual ligament had also progressed significantly. From the aforementioned results, it was confirmed that the transplanted artificial tissue body for ligament/tendon had matured into a ligament *in vivo.*

### [Example 2]

A recombinant human fibroblast growth factor solution, a recombinant human GDF-7 solution, a recombinant human CTGF solution, a recombinant human TGF-β3 solution, a medium, and cells were the same as those used in Example 1.

### [Production of artificial tissues]

< Cells >

### (Example 2-1) Production of meniscus

NHDFs were seeded at a density of 8 x 10⁵ cells/dish on a 150 mm culture dish (FALCON, 353025), were then cultured for 1 week using a fibroblast culture kit, and were then harvested. IPSC-MSCs were seeded at a density of 1 x 10⁶ cells/dish on a 150 mm culture dish (FALCON, 353025), were then cultured in an α-MEM medium for 1 week, and were then harvested. IPSC-SCLs were harvested on the 19th day after initiation of induction of meniscus progenitor cells from iPS cells. The cells were mixed at a ratio of 10% IPSC-SCLs, 45% NHDFs, and 45% IPSC-MSCs, and were then suspended in a medium for structure production. Thereafter, the suspension was seeded in a 96-well plate (Sumitomo Bakelite Co., Ltd., MS-9096U) at a density of 3.5 x 10⁴ cells/well to form spheroids. Two days later, the spheroids were laminated on one another using a three-dimensional cell lamination system (Cyfuse, Regenova). Ten layers of spheroids were laminated on one another layered with intervals of 500 µm on a 7 mm diameter round *Kenzan* (Cyfuse, NA1029). After 7 days of culture with the medium for construct production circulated by a peristaltic pump, a laminate was extracted from the round *Kenzan* to obtain a circular three-dimensional tissue body.

Stretch stimulation was performed on the three-dimensional tissue body in the stretch step. Two metal rods were inserted into the cavity of the circular three-dimensional tissue body, which was placed in a device. and was then stretched. Stretching was initiated on the 7th day after the tissue body had been removal from the *Kenzan*, and the stretching was performed at a stretch percentage of 5%, a stretch number of 0.5 Hz, and a stretch time of 30 minutes per day. Stretch stimulation was applied for 5 consecutive days, followed by no stretching for 2 days, and this operation was repeated.

Meniscus differentiation-inducing factors were added in the stretch step of the three-dimensional tissue body. A BMP-12 solution, a CTGF solution, and a TGF-β3 solution were added at a concentration of 10 ng/ml each. The solutions were added one month after the tissue body had been removed from the *Kenzan*, and the obtained mixture was then cultured for one week.

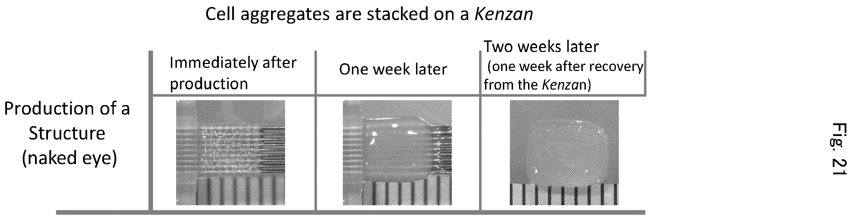

The state of culture using this *Kenzan* is shown in Figure 21. In Figure 21, photographs of cell aggregates immediately after lamination on the *Kenzan*, the cell aggregates one week later, and the cell aggregates two weeks later are shown from the left. From the obtained results, it was confirmed that tightly cohesive cell aggregates were formed as the culture progressed.

### Immunohistochemical staining

The three-dimensional structure obtained above was fixed in 10% neutral buffered formalin, was then dehydrated and/or degreased, and was then thinly sectioned into paraffin blocks. As an antibody, rabbit anti-human MKX (ATLAS ANTIBODIES, HPA006927) or rabbit anti-human SCXA (abcam, ab58655) was used, and was reacted overnight at 4°C. Coloring was performed using a DAB polymer method.

As a result of the present experimental conditions, as in Figure 10, since the presence of Mkx-positive cells, Scx-positive cells, and further, Tenm-positive cells was certainly confirmed, it was confirmed that the three-dimensional tissue body according to the present Example 2-1 has the function of meniscus.

### (Example 2-2) Confirmation of stretch stimulation

The presence or absence of stretch stimulation on the three-dimensional tissue body in the culture step was confirmed.

In the same manner as the operation shown in Figure 11, two metal rods were inserted into the three-dimensional tissue body that was a sheet-shaped structure, and this three-dimensional tissue body was then placed in the device (automatic stretching culture vessel) shown in Figure 1 and was stretched.

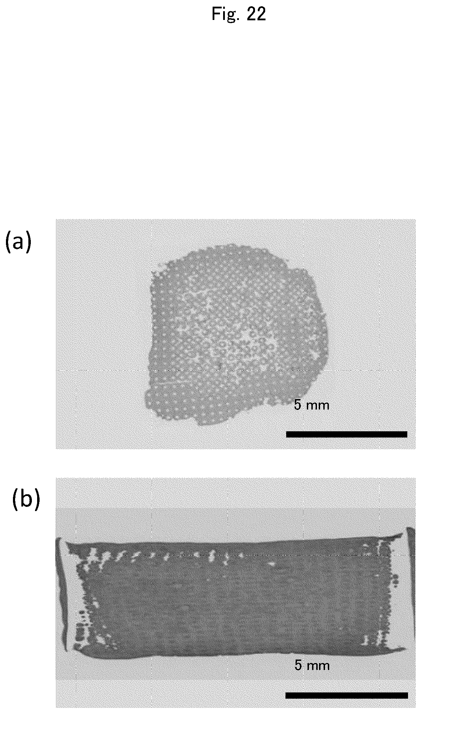

Type I collagen immunostaining was confirmed using an optical microscope. The results of the optical microscope image of the three-dimensional tissue body before the stretch stimulation are shown in Figure 22(a). From these results, it was confirmed that type I collagen immunostaining was not observed in the absence of stretch stimulation. On the other hand, the results of the optical microscope image of the three-dimensional tissue body after the stretch stimulation are shown in Figure 22(b). From these results, it was confirmed that type I collagen immunostaining was observed as a result of the stretch stimulation.

Next, the orientation of type I collagen was confirmed using a polarizing microscope. The results of the polarizing microscope image of the three-dimensional tissue body before the stretch stimulation were the same as those in Figure 13. That is to say, in the absence of stretch stimulation, there was confirmed a structure, in which the orientation of type I collagen was not aligned and individual cell masses were randomly present. In addition, the results of the polarizing microscope image of the three-dimensional tissue body after the stretch stimulation was also the same as those in Figure 13. That is, it was confirmed that there was obtained a structure, in which the orientation of type I collagen was aligned in a certain direction by stretch stimulation.

### (Example 2-3) Confirmation of circulating culture

The three-dimensional tissue body in the stretch step was confirmed in the presence or absence of circulating culture. In the same manner as that of Example 1-4, as shown in Figure 14, after cell masses had been laminated on a tube, the laminate was placed in the device (automatic stretching culture vessel) shown in Figure 1, and a tensile test was performed to confirm a difference in the presence or absence of a tube pump 5, namely, a difference between the absence of the tube pump 5 such as a peristaltic pump (registered trademark) that enables circulating culture in the vessel (static culture) as shown in Figure 14(a), and the presence of the peristaltic pump connecting to a tube (circulating culture) as shown in Figure 14(b).

### Tensile test

A force gauge (Imada, ZTA-500N) was attached to a motorized measuring stand (Imada, MX2-500N-FA), and the measurement was carried out. Two metal rods were passed through the lumen of the three-dimensional tissue body, one of which was fixed to the stand, and the other was passed through the force gauge. The force gauge side was pulled at a speed of 50 mm/min, and the load at which the three-dimensional tissue body was broken was examined.

Regarding breaking load [mN], as the results of the static culture, it was confirmed that the circulating culture exhibited more than two or more times the strength than the static culture, as with Example 1.

Moreover, regarding rigidity [N/mm] as well, it was confirmed that the circulating culture exhibited more than two or more times the strength than the static culture, as with Example 1.

### (Example 2-4) Confirmation of compressed culture

The effects obtained by compressive stimulation in the compression step of the three-dimensional tissue body were confirmed.

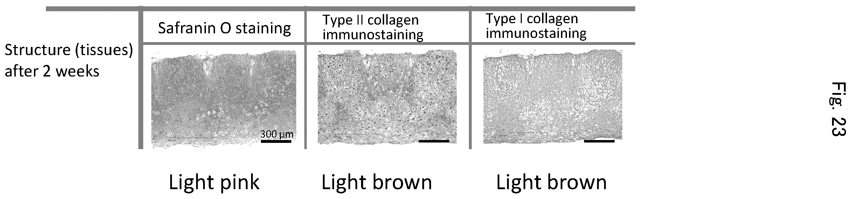

After completion of the above-described stretch step, compressive stimulation was applied to the three-dimensional tissue body, using the above-mentioned device of Figure 5 (automatic compression culture vessel 1B), and the tissue body was cultured. After that, the three-dimensional tissue body was confirmed by subjecting to safranin O staining, type I collagen immunostaining, and type II collagen immunostaining. The obtained staining results are shown in Figure 23. As shown in Figure 23, in the central portion of the three-dimensional tissue body to which compressive stimulation was applied (i.e., the portion that constitutes the inner circumference lateral side portion of the meniscus tissue body), it was confirmed that the orientation of type II collagen was certainly aligned, and further, an increase in proteins that hold water molecules was confirmed by coloring with safranin O staining. Thus, it was confirmed that the central portion of the three-dimensional tissue body exhibits properties similar to the cartilage possessed by the meniscus.

### (Example 2-5) Confirmation of raw material cells

The types of raw material cells in the mixing step were confirmed.

Two types of raw material cells were examined; namely, a case where iPS cell-derived mesenchymal stem cells were used as raw material cells with respect to fibroblasts, and a case where somatic stem cells were used as raw material cells.

Regarding breaking load [mN], the results of a case where fibroblasts and somatic stem cells were used (with stretch stimulation) were the same as those shown in Figure 17. The composition ratio between the fibroblasts and the somatic stem cells was 1 : 1. In addition, the results of a case where fibroblasts and somatic stem cells were used (without stretch stimulation) were also the same as those shown in Figure 17. The composition ratio between the fibroblasts and the somatic stem cells was 1 : 1. Moreover, the results of a case where fibroblasts and iPS cell-derived mesenchymal stem cells were used (with stretch stimulation) were also the same as those shown in Figure 17. The composition ratio between the fibroblasts and the iPS cell-derived mesenchymal stem cells was 1 : 1.

As a result, it was confirmed that when the fibroblasts and the iPS cell-derived mesenchymal stem cells were used (with stretch stimulation), the breaking load [mN] exceeded the detection limit value, and a stronger strength was obtained in the use of the fibroblasts and the iPS cell-derived mesenchymal stem cells than when the fibroblasts and the somatic stem cells were used.

### [Example 3]

A recombinant human fibroblast growth factor solution, a recombinant human GDF-7 solution, a recombinant human CTGF solution, a recombinant human TGF-β3 solution, a medium, and cells were the same as those used in Example 1.

In Example 3, a transplant for meniscus (band-shape) was produced, and a transplantation test was carried out to transplant it into a pig.

### < Method >

The medial condyle of the femur of a pig was excised and inverted from the distal end of the femur without damaging the medial collateral ligament, so as to expose the medial meniscus. Next, bone holes were each created at the attachment points of the anterior and posterior roots of the medial meniscus at the upper edge of the tibia and at the location connecting the medial surface of the upper end of the tibia. After the medial meniscus had been completely excised, the sutures that were sutured to both ends of the transplant for meniscus were passed through individual bone holes, and the sites corresponding to the anterior, middle, and posterior segments of the transplant for meniscus were sutured to the joint capsule. The sutures that were passed through the bone holes were then fixed on the medial surface of the upper end of the tibia. After that, the femur medial condyle was returned to its original position, and the femur medial condyle was fixed to the distal part of the femur with a screw.

### < Results >

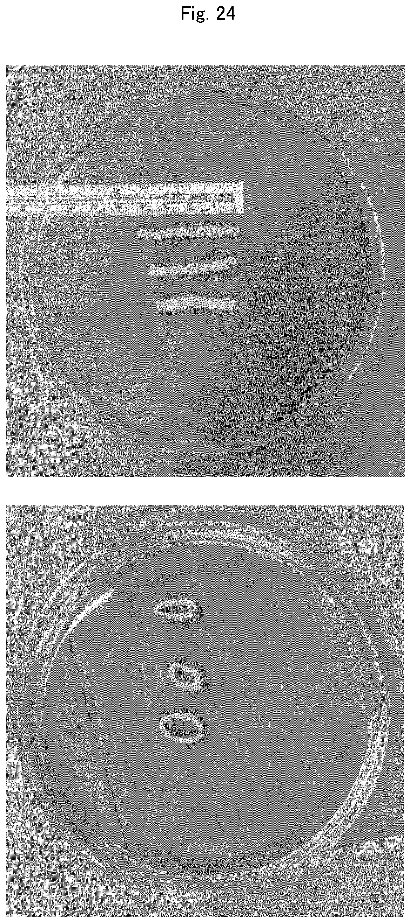

The results are shown in Figures 24 to 30.

Figure 24 is a view showing ring-shaped and band-shaped artificial tissue bodies for meniscus, and by cutting and spreading out a portion of the ring-shaped artificial tissue body for meniscus, it was possible to obtain a band-shaped artificial tissue body for meniscus.

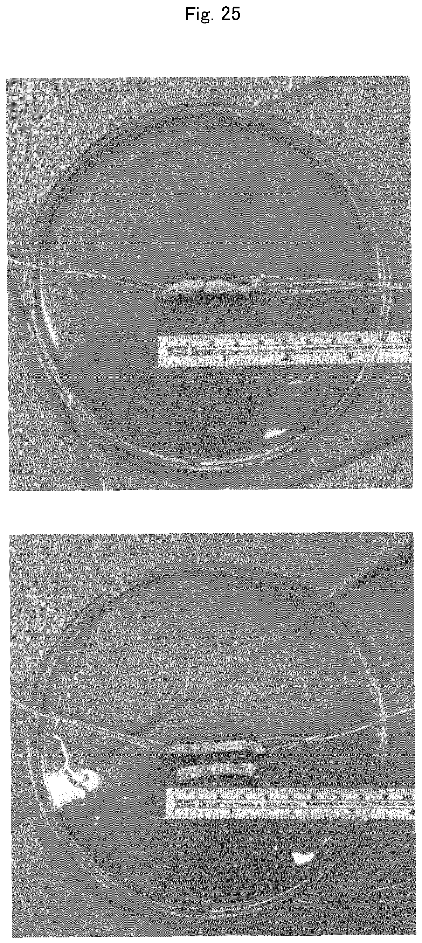

Figure 25 is a view showing one transplant for meniscus produced by bundling and suturing three band-shaped artificial tissue bodies for meniscus, and it was possible to apply tension in opposite directions to the sutures at both ends.

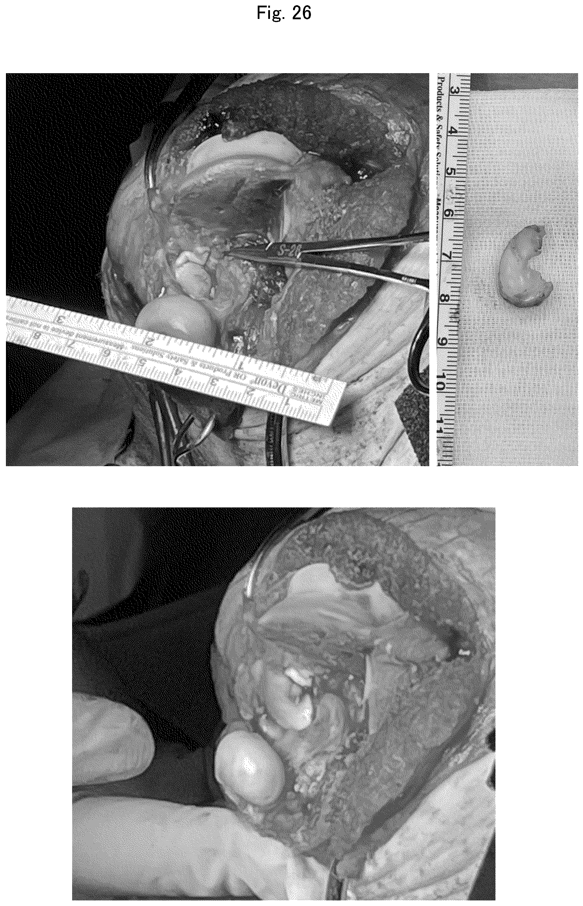

Figure 26 is a view showing that the medial condyle of the femur of a pig was cut and inverted to expose the medial meniscus, which was then completely excised, and it was possible to perform a total meniscus replacement on a pig's knee joint without damaging the ligament.

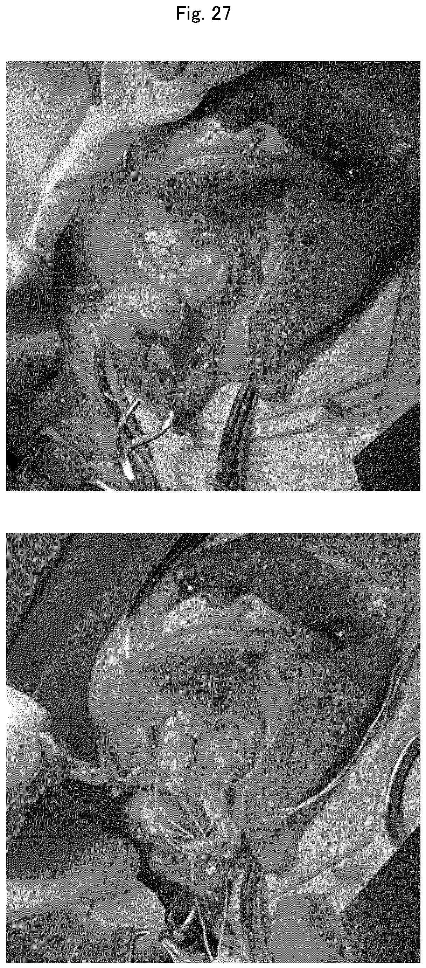

Figure 27 is a view showing that a suture was passed through a bone hole created in the tibia and a transplant for meniscus was placed on the upper edge of the tibia, and it was possible to perform a total meniscus replacement by transplanting the meniscus in a crescent shape similar to that of a healthy meniscus.

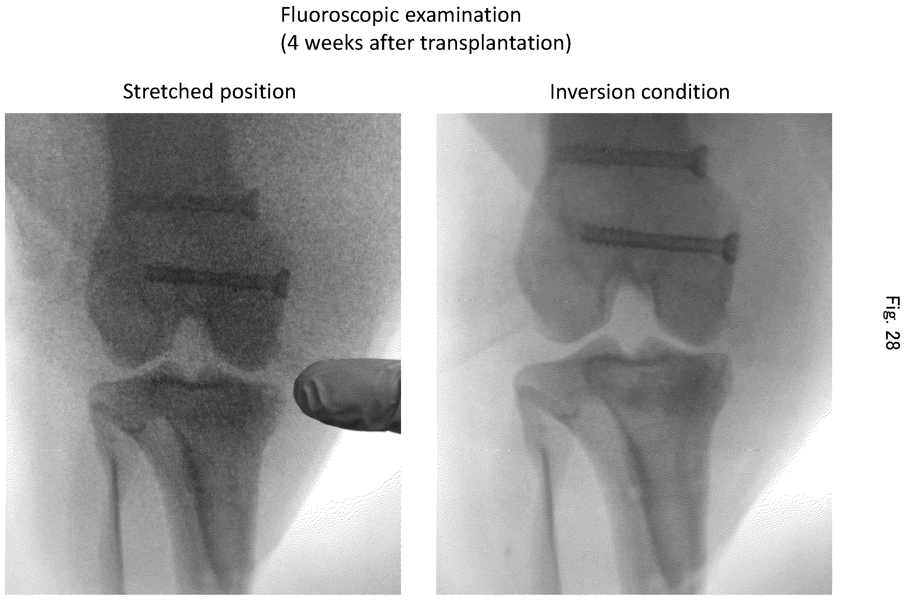

Figure 28 is a view showing the results of a fluoroscopic examination of a pig's knee joint four weeks after meniscus transplantation, and it was confirmed that the transplant for meniscus played a role as a shock absorber in the knee joint, while maintaining the shape of a meniscus, without narrowing the joint space.

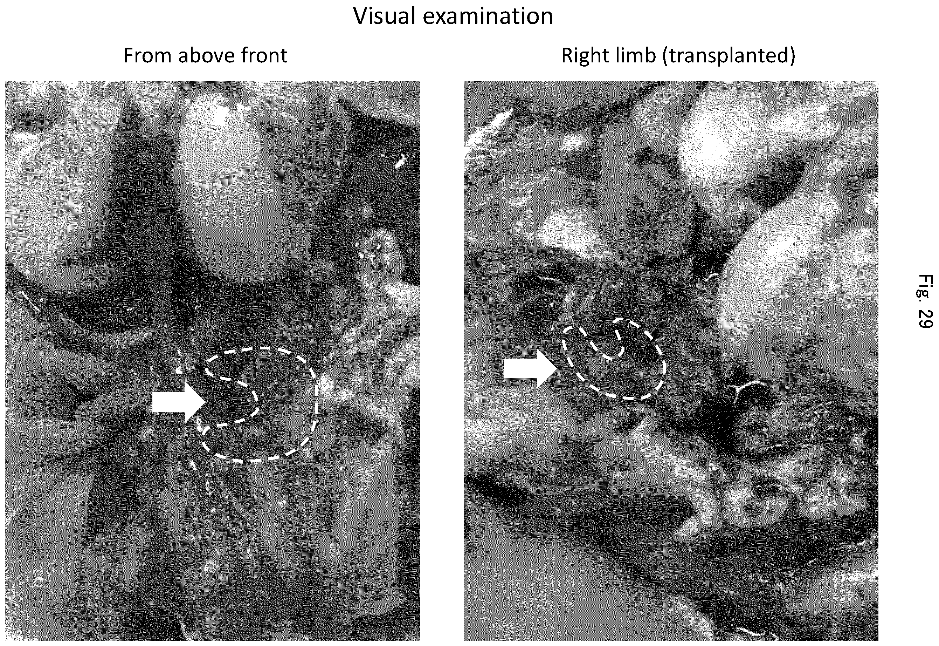

Figure 29 is a view showing the results of a visual examination of a pig's knee joint when it was incised, and the presence of a transplant for meniscus with a shape similar to that of a meniscus was confirmed.

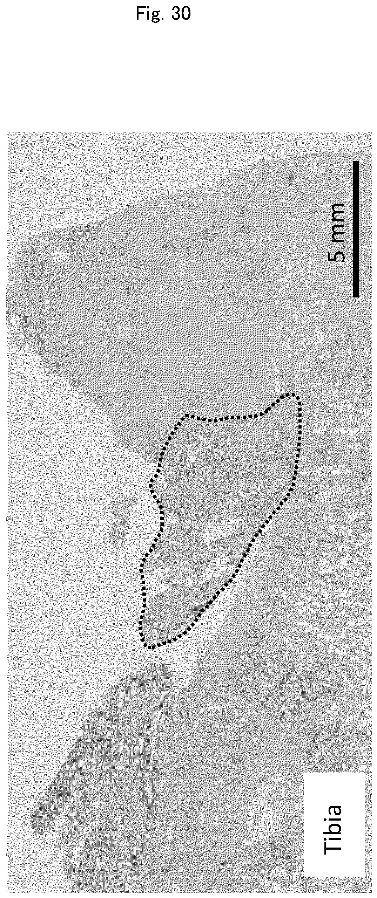

Figure 30 is a view showing the results of a histological examination of a transplant for meniscus 4 weeks after the transplantation of meniscus, and the tissue morphology similar to that of a meniscus was confirmed.

## Claims

1. A method for enhancing the strength of a three-dimensional tissue body, comprising a step of culturing a three-dimensional tissue body formed by stacking cell masses, while applying stretch stimulation and/or compressive stimulation to the three-dimensional tissue body.

2. The method according to claim 1, wherein the stretch stimulation is stretch stimulation including uniaxial movement or multiaxial movement.

3. The method according to claim 2, wherein the stretch stimulation includes at least one movement from among twisting movement, tensile movement and vibration movement.

4. The method according to claim 1, wherein the compressive stimulation is performed by applying pressure to the three-dimensional tissue body using a pressing unit having a pressing surface.

5. The method according to claim 1, wherein a medium for culturing the three-dimensional tissue body further comprises an additive factor.

6. The method according to claim 1, wherein the three-dimensional tissue body is cultured under circulating culture.

7. The method according to claim 1, wherein the cell masses comprise mesenchymal stem cells, fibroblasts, and cells that are raw materials for a tissue body of interest.

8. The method according to claim 7, wherein the cells that are raw materials are progenitor cells of bone, cartilage, ligament, tendon or meniscus.

9. The method according to claim 8, wherein the progenitor cells are sclerotome cells.

10. The method according to claim 9, wherein the three-dimensional tissue body is a tissue body for use in bone, cartilage, ligament, tendon or meniscus.

11. A method for producing a three-dimensional tissue body with an enhanced strength, wherein the method comprises a step of culturing a three-dimensional tissue body formed by stacking cell masses, while applying stretch stimulation and/or compressive stimulation to the three-dimensional tissue body.

12. The method according to claim 11, wherein the stretch stimulation is stretch stimulation including uniaxial movement or multiaxial movement.

13. The method according to claim 11, wherein the stretch stimulation includes at least one movement from among twisting movement, tensile movement and vibration movement.

14. The method according to claim 11, wherein the compressive stimulation is performed by applying pressure to the three-dimensional tissue body using a pressing unit having a pressing surface.

15. The method according to claim 11, wherein a medium for culturing the three-dimensional tissue body further comprises an additive factor.

16. The method according to claim 11, wherein the three-dimensional tissue body is cultured under circulating culture.

17. The method according to claim 12, wherein the cell masses comprise mesenchymal stem cells, fibroblasts, and cells that are raw materials for a tissue body of interest.

18. The method according to claim 17, wherein the cells that are raw materials are progenitor cells of bone, cartilage, ligament, tendon or meniscus.

19. The method according to claim 18, wherein the progenitor cells of bone, cartilage, ligament, tendon or meniscus are sclerotome cells.

20. The method according to claim 19, wherein the three-dimensional tissue body is a tissue body for use in bone, cartilage, ligament, tendon or meniscus.

21. A three-dimensional tissue body having a breaking strength of at least 0.001 N/mm, which is prepared by culturing a three-dimensional tissue body formed by stacking cell masses, while applying stretch stimulation and/or compressive stimulation to the three-dimensional tissue body.

22. The tissue body according to claim 21, wherein the cell masses comprise mesenchymal stem cells, fibroblasts, and cells that are raw materials for a tissue body of interest.

23. The tissue body according to claim 22, wherein the cells that are raw materials are progenitor cells of bone, cartilage, ligament, tendon or meniscus.

24. The tissue body according to claim 23, wherein the progenitor cells of bone, cartilage, ligament, tendon or meniscus are sclerotome cells.

25. The tissue body according to claim 24, wherein the three-dimensional tissue body is a tissue body for use in bone, cartilage, ligament, tendon or meniscus.

26. A culture device for culturing a three-dimensional tissue body, including an application unit for applying mechanical stimulation to the three-dimensional tissue body.

27. The culture device according to claim 26, wherein
the mechanical stimulation applied by the application unit includes at least stretch stimulation.

28. The culture device according to claim 26, wherein
the three-dimensional tissue body has an opening,
the application unit includes at least two shaft members, and
the application unit relatively moves the at least two shaft members in a state in which the at least two shaft members are inserted into the opening, so as to apply mechanical stimulation to the three-dimensional tissue body.

29. The culture device according to claim 28, wherein
the application unit relatively translationally moves the at least two shaft members, so as to apply mechanical stimulation to the three-dimensional tissue body.

30. The culture device according to claim 28, wherein
the application unit relatively rotationally moves the at least two shaft members, so as to apply mechanical stimulation to the three-dimensional tissue body.

31. The culture device according to claim 26,
the application unit includes a vibrator that generates vibrations, and
the application unit stretches the three-dimensional tissue body with the vibrations generated by the vibrator, so as to apply mechanical stimulation to the three-dimensional tissue body.

32. The culture device according to claim 26, wherein
the mechanical stimulation applied by the application unit includes at least compressive stimulation.

33. The culture device according to claim 26, wherein
the application unit includes
a pedestal unit having a recess formed therein, into which the three-dimensional tissue body is fitted, and
a pressing unit for pressing the three-dimensional tissue body fitted into the recess.

34. The culture device according to claim 26, wherein
the application unit includes
a tensile unit for pulling the three-dimensional tissue body, and
a pressing unit that presses the three-dimensional tissue body, to which stretch stimulation has been applied by the tensile unit, in a direction different from the tensile direction of the tensile unit.

35. The culture device according to claim 26, which further includes
a circulation unit for circulating a culture medium in a culture vessel.

36. The culture device according to claim 26, which further includes
a gas regulation unit for regulating gas in a culture vessel.

37. The culture device according to claim 26, which further includes
a temperature regulation unit for regulating temperature in a culture vessel.

38. The culture device according to claim 26, which further includes
a detection unit for detecting a physical quantity corresponding to the size of the mechanical stimulation applied to the three-dimensional tissue body by the application unit, and
a control unit for controlling the size of the mechanical stimulation applied by the application unit, based on the detection results by the detection unit.
